(19) Europäisches Patentamt
European Patent Office
Office européen des brevets

(11) Veröffentlichungsnummer: **0 328 160 B1**

(12) **EUROPÄISCHE PATENTSCHRIFT**

(45) Veröffentlichungstag der Patentschrift: **04.05.94**

(51) Int. Cl.$^5$: **C07K 5/06**, A61K 37/64

(21) Anmeldenummer: **89105371.2**

(22) Anmeldetag: **22.08.81**

(60) Veröffentlichungsnummer der früheren Anmeldung nach Art. 76 EPÜ: **0 046 953**

# (54) Aminosäurederivate, Verfahren zu ihrer Herstellung, diese enthaltende Mittel und deren Verwendung.

(30) Priorität: **30.08.80 DE 3032709**
**08.05.81 DE 3118191**

(43) Veröffentlichungstag der Anmeldung:
**16.08.89 Patentblatt 89/33**

(45) Bekanntmachung des Hinweises auf die Patenterteilung:
**04.05.94 Patentblatt 94/18**

(84) Benannte Vertragsstaaten:
**AT BE CH DE FR GB IT LI LU NL SE**

(56) Entgegenhaltungen:
**EP-A- 0 012 845**
**EP-A- 0 024 852**

**Keine Entgegenhaltungen.**

(73) Patentinhaber: **HOECHST AKTIENGESELLSCHAFT**

**D-65926 Frankfurt(DE)**

(72) Erfinder: **Geiger, Rolf, Prof. Dr.**
**Heinrich-Bleicher-Strasse 33**
**D-6000 Frankfurt am Main 50(DE)**
Erfinder: **Teetz, Volker, Dr.**
**An der Tann 20**
**D-6238 Hofheim am Taunus(DE)**
Erfinder: **Urbach, Hansjörg, Dr.**
**Le Lavandoustrasse 41**
**D-6242 Kronberg/Taunus(DE)**
Erfinder: **Schölkens, Bernward, Dr.**
**Hölderlinstrasse 62**
**D-6233 Kelkheim (Taunus)(DE)**
Erfinder: **Henning, Rainer, Dr.**
**Rotenhofstrasse 31**
**D-6234 Hattersheim am Main(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 328 160 B1

Rank Xerox (UK) Business Services
(3.10/3.09/3.3.3)

## Beschreibung

Die Erfindung betrifft eine Verbindung der Formel I

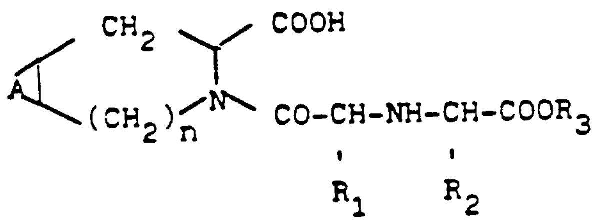

(I)

in welcher bedeutet:
n = 0 und

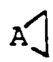

einen Benzolring,

$R_1$ und $R_2$, die gleich oder verschieden und auch ihrerseits substituiert sein können, je
- Alkyl oder Alkenyl mit bis zu 6 C-Atomen,
- Cycloalkyl oder Cycloalkenyl mit je 5 bis 7 C-Atomen,
- Aryl oder teilhydriertes Aryl mit 6 bis 10 C-Atomen,
- ein mono- oder bicyclischer Heterocyclus mit 5 bis 7 bzw. 8 bis 10 Gliedern, davon 1 bis 2 -S- oder -O- und/oder bis zu 4 -N-Atomen,

$R_3$ Wasserstoff, Alkyl mit 1 bis 6 C-Atomen, Alkenyl mit 2 bis 6 C-Atomen oder Aralkyl mit 7 bis 14 C-Atomen.

und deren physiologisch verträgliche Salze.

Als bevorzugte Beispiele für $R_1$ und $R_2$ seien insbesondere genannt, Methyl, Ethyl, n-Propyl, Isopropyl, n-Butyl, Isobutyl, tert.-Butyl, sowie die geradkettigen und verzweigten Pentyle und Hexyle, ferner Cyclopentyl, Cyclohexyl, Cycloheptyl, Cyclopentenyl, Cyclohexenyl oder Cycloheptenyl, Phenyl, Naphthyl, Di- und Tetrahydronaphthyl, Benzyl, Phenethyl, p-Fluorphenethyl, o-Methylphenethyl, p-Methoxyphenethyl, 2,4-Dichlorphenethyl, Cyclohexyläthyl.

Die 5- bis 7-gliedrigen mono- oder 9-, 10-gliedrigen bicyclische Heterocyclen können unsubstituiert sein, aber auch einen oder mehrere gleiche oder verschiedene Substituenten tragen wie Halogen, Sauerstoff (auch Sulfoxid oder Sulfon), Hydroxy, Carboxy, Carbonamido, Sulfonamido, Nitro, Alkyl und Aralkyl mit bis zu 9 C-Atomen, Methoxy oder Ethoxy. Im Falle einer Substitution ist Mono- oder Disubstitution bevorzugt.

Als bevorzugte Bedeutungen für $R_3$ seien genannt:
Wasserstoff, Ethyl, Butyl, t-Butyl, Benzyl, p-Nitrobenzyl.

Die als $R_1$ oder $R_2$ infragekommenden Alkyle oder Alkenyle können geradkettig oder verzweigt sein. Sie können einen oder mehrere gleiche oder verschiedene Substituenten tragen, insbesondere:

Cycloalkyl oder Cycloalkenyl mit 5-7 C-Atomen, Hydroxy Alkoxy mit 1 bis 2 C-Atomen, Aryloxy mit 1 bis 2 C-Atomen im Alkylteil, wobei der Alkylteil durch Methoxy, Ethoxy, Carboxy, Carbonamido, Amino oder Alkylamino und der Arylteil durch die genannten Substituenten und zusätzlich durch Halogen oder Nitro substituiert sein kann, Amino,

Mono-, Di- oder Trialkyl- oder -cycloalkyl-amino mit bis zu insgesamt 7 C-Atomen in den Alkyl- oder Cycloalkylgruppen, das gegebenenfalls durch Hydroxy, Carboxy, Carbonamido, Carbethoxy, Amino, Alkyl- oder Dialkylamino, Piperidino oder Morpholino im Alkylrest substituiert sein kann,

Alkyl-, Aryl-, Aralkyl-oxycarbonylamino oder -ureido, Formyl, Alkanoyl-, Aroyl- oder Aralkanoylamino mit bis zu 10 C-Atomen, Arylamino, Aralkylamino, in denen der Arylteil durch Alkyl oder Alkoxy mit 1 bis 2 C-Atomen, Methylendioxy, Amino, Hydroxy, Acetoxy, Carboxy, Carbonamido, Carbethoxy, Halogen, oder Nitro mono- oder disubstituiert sein kann,

Alkyl-, Aryl- oder Aralkylmercapto mit bis zu 7 C-Atomen, wobei der Alkylteil durch Methoxy, Ethoxy, Hydroxy, Carboxy, Carbonamido, Carbethoxy, Amino oder Alkylamino und der Arylteil durch die genannten Reste und zusätzlich durch Halogen, Nitro oder Sulfonamido substituiert sein kann, ferner deren Sulfoxide und Sulfone,

Carboxy, Carbethoxy, Carbonamido, Alkyl-, Cycloalkyl-, Cycloalkylen- oder Dialkylaminocarbonyl mit bis zu 6 C-Atomen, Aryl- oder Aralkylaminocarbonyl,

Guanido,

Phenyl, Naphthyl, Di- und Tetrahydronaphthyl, das durch Halogen, Hydroxy, Acetoxy, Carboxy, Carbonamido, Sulfonamido, Nitro, Methyl, Ethyl, Methoxy oder Ethoxy mono- oder disubstituiert sein kann,

5 bis 7-gliedrige mono- oder 9- bis 10-gliedrige bicyclische Heterocyclen, gegebenenfalls 1 bis 2 S- oder O-Atome und/oder bis 4 N-Atome pro Ring enthaltend, die gegebenenfalls wie oben substituiert sein können.

Unter Alkyl Ist, falls Im Einzelfall nichts anderes gesagt, vorzugsweise ein geradkettiges oder verzweigtes Alkyl mit 1 bis 6 C-Atomen zu verstehen. Gleiches gilt für Alkanoyl, Alkylamino, Alkylmercapto.

Aryl bedeutet bevorzugt Phenyl, durch Halogen, Alkyl oder Alkoxy substituiertes Phenyl oder Naphthyl. Gleiches gilt für Aroyl, Arylamino, Arylmercapto.

Aralkyl umfaßt bevorzugt Benzyl, Phenethyl und die entsprechenden, im Phenylkern mit Halogen, Nitro, Alkyl oder Alkoxy substituierten Verbindungen. Gleiches gilt für Aralkanoyl, Aralkylamino, Aralkylmercapto.

Verbindungen der allgemeinen Formel I, in der $R_1$ die Seitenkette einer natürlich vorkommenden L-Aminosäure darstellt, z.B. Methyl, Isobutyl, Methylthioethyl, Carboxymethyl, Carboxyethyl, Amino-n-butyl, Guanido-n-propyl, Imidazol-4-ethyl, Benzyl, 4-Hydroxybenzyl oder 3-Indolmethyl, sowie deren funktionelle Derivate wie Ether, Ester oder Amide sind besonders bevorzugt. Der Rest $R_1$ kann aber auch Teil von Mercapto-, Hydroxy- oder Aminosäuren sein, die nicht in der Natur vorkommen.

Gegenstand der Erfindung ist ferner ein Verfahren zur Herstellung der Verbindungen der Formel I, das dadurch gekennzeichnet ist, daß man

(a) eine Verbindung der Formel II mit einer Verbindung der Formel III umsetzt

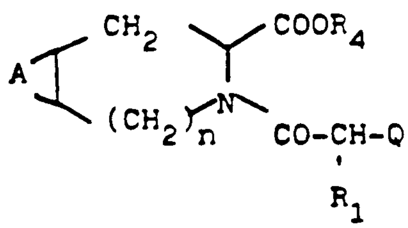

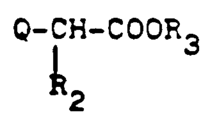

II

III

in denen ein Q eine nucleofuge Gruppe und das andere Q $-NH_2$ bedeutet und $R_4$ für H, Methyl, Ethyl, Benzyl oder tert.-Butyl steht, und gegebenenfalls einen erhaltenen Ester in die Carbonsäure ($R_3$ und/oder $R_4$ = Wasserstoff) überführt, oder

(b) eine Verbindung der Formel IV

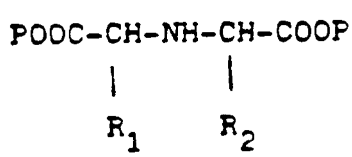

IV

worin P H bedeutet, eines der beiden P jedoch auch die Bedeutung von $R_3$ oder tert.-Butyl haben kann, mit einer Verbindung der Formel V

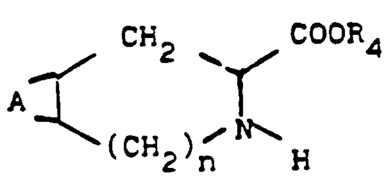

V

in Gegenwart eines Kondensationsmittels umsetzt, und gegebenenfalls eine oder beide Estergruppen in

die Carbonsäure überführt, oder

(c) eine Verbindung der Formel VI mit einer Verbindung der Formel VII umsetzt

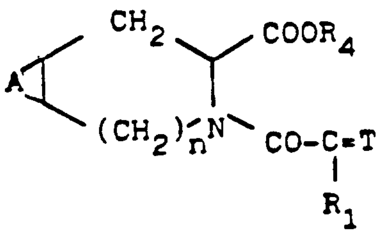

VI

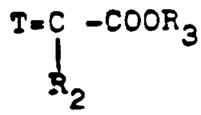

VII

in denen ein T für eine $NH_2$-Gruppe und das andere T für ein Sauerstoffatom stent, und die erhaltene Schiffsche Base reduziert.

Zur Herstellung der Verbindungen I kann man beispielsweise von 2-Halogencarbonsäurederivaten II oder III ausgehen, die mit Nucleophilen reagieren. Diese Reaktion wird bevorzugt in mit Wasser mischbaren organischen Lösungsmitteln, gegebenenfalls in Anwesenheit von Wasser, durchgeführt, wobei eine anorganische oder tertiäre oder quartäre organische Base zugegeben wird. Wenn die Reaktionspartner in aprotischen organischen Lösungsmitteln löslich sind, ist ein solches Lösungsmittel vorzuziehen, wobei als Base vorteilhaft ein Trialkylamin, Tetraalkylammoniumhydroxyd oder Tetramethylguanidin oder eine Suspension eines Alkali- oder Erdalkalicarbonats zugesetzt werden.

Die Ausgangsprodukte

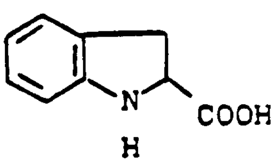

VIII

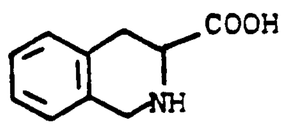

IX

sind literaturbekannt, ihre spezifische pharmakologische Wirkung als Bestandteil der erfindungsgemäßen Verbindungen der Formel I wurde jedoch bisher nicht beschrieben. Die Verbindung VIII kann nach Aust. J. Chem. 20 (1967), Seite 1935, und die Verbindung IX nach J. Amer. Chem. Soc. 70 (1948), Seite 182 hergestellt werden.

Die Ausgangsprodukte

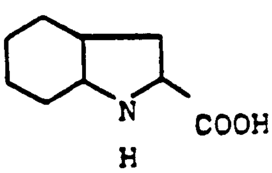

X

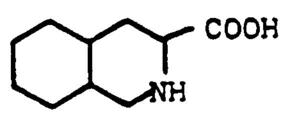

XI

werden durch katalytische Hydrierung, bevorzugt an Rhodiumkatalysatoren, aus den Verbindungen der Formel VIII bzw. IX hergestellt.

Aus innen erhält man die Ester mit $R_4$ in bekannter Weise und kondensiert sie mit Halogencarbonsäuren zu den Verbindungen II (Q = Halogen) entweder über die Säurechloride, gemischte Anhydride, Aktivester oder andere Methoden, wie sie in Houben-Weyl, Methoden der Organischen Chemie, Band 15, ausführlich beschrieben sind. Bei $R_1$ = $CH_3$ kann man z.B. von der aus L-Milchsäure leicht zugänglichen D-2-Chlorpropionsäure ausgehen.

Die Zwischenprodukte der Formel II (Q = $NH_2$) werden aus den Carbonsäureestern der allgemeinen Formel V durch Kondensation mit einer N-geschützten 2-Aminocarbonsäure erhalten. Die Schutzgruppe

wird nach beendeter Kondensation wieder abgespalten. Als Schutzgruppe kommt z.B. Benzyloxycarbonyl in Frage.

Die Kondensation kann nicht nur mit DCC/HOBt (Dicyclohexylcarbodiimid/1-Hydroxybenzotriazol) sondern auch mit einem anderen gegeigneten Kondensationsmittel z.B. dem in Houben-Weyl, Band 15 beschriebenen, durchgeführt werden.

Die Verfahrensweise (b) geht von Verbindungen der Formel IV aus.

Sind $R_1$ und $R_2$ identisch, so kann P Wasserstoff sein. Man kondensiert dann z.B. in Gegenwart von Dicyclohexylcarbodiimid, mit einem Äquivalent einer Verbindung der Formel V und spaltet $R_4$ in bekannter Weise ab. Die Reaktion verläuft möglicherweise über das isolierbare Anhydrid XII, das anschließend mit einer Verbindung der Formel V geöffnet wird.

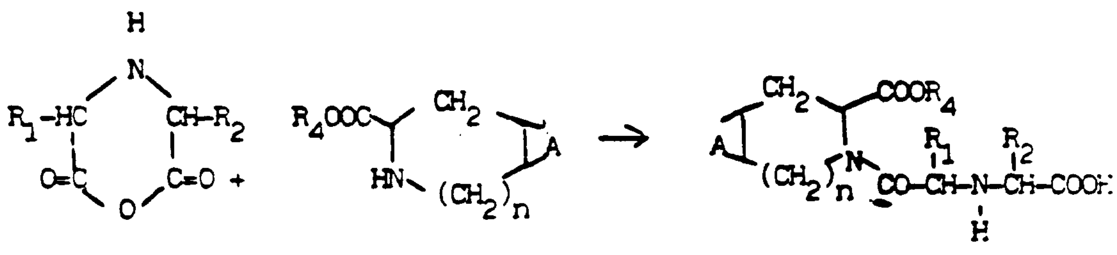

XII. V

Sind die Reste $R_1$ und $R_2$ in der Verbindung IV ungleich, so ist vorzugsweise nur einer der Reste P Wasserstoff, der andere sollte vorzugsweise die Bedeutung von $R^3$ haben oder tert.-Butyl sein. Man kondensiert dann in beschriebener Weise mit den Verbindungen der Formel V und überführt gegebenenfalls eine oder beide Estergruppen in die Carbonsäure.

Die für T = $NH_2$ in den Formeln VI bzw. VII bedeutsame Kondensation mit 2-Ketocarbonsöuren bzw. deren Estern gemäß (c) führt in an sich bekannter Weise über Schiff'sche Basen z.B. nach Reduktion mit Natriumborhydrid oder Natriumcyanoborhydrid, durch katalytische Hydrierung oder elektrolytische Reduktion zu den entsprechenden Verbindungen der Formel I in guter Reinheit.

Die neuen Verbindungen besitzen unter anderem 3 chirale Zentren an $\alpha$-C-Atomen. Die Anordnung der Liganden entspricht bevorzugt der L-Konfiguration. Im allgemeinen können durch Kristallisation sterisch weitgehend einheitliche Verbindungen der Formel I erhalten werden. Bevorzugt sind Gegenstromverteilung oder präparative HPLC zur Anreicherung sterisch einheitlicher Formen.

Auf der Stufe der Zwischenprodukte X und XI kann man gegebenenfalls Diastereomere durch Kristallisation oder präparative HPLC trennen, so daß nach dem oben beschriebenen Verfahren Produkte der Formel I erhalten werden können, die an allen chiralen Zentren eine einheitliche sterische Anrodnung besitzen.

Aus EP-A 012 845 sind Tetrahydroisoquinoline mit einer Mercapo-Seitenkette bekannt, die als Arzneimittel Einsatz finden können. Drüber hinaus werden in der prioritätsälteren, nicht vorveröffentlichten EP-A 024 852 Indolin-Derivate vorgestellt.

Die neuen Verbindungen der Formel I besitzen eine langdauernde, intensive blutdrucksenkende Wirkung.

Sie können zur Bekämpfung des Bluthochdrucks verschiedener Genese eingesetzt und für sich oder in Kombination mit anderen blutdrucksenkenden, gefäßerweiternden oder diuretisch wirksamen Verbindungen angewandt werden. Typische Vertreter dieser Wirkklassen sind z.B. in Erhart-Ruschig, Arzneimittel, 2. Auflage, Weinheim 1972, beschreiben. Die Anwendung kann intravenös, subcutan oder peroral erfolgen.

Die Dosierung bei peroraler Gabe liegt bei 20 - 200 mg je Einzeldosis. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden. Bei intravenöser oder subcutaner Verabreichung sollte die Einzeldosis zwischen 0,01 und 10 mg liegen.

Die Verbindungen der Formel I liegen als innere Salze vor. Falls beide Carboxylgruppen frei sind, können zusätzlich Alkali- und Erdalkalisalze und solche mit physiologisch unbedenklichen Aminen gebildet werden. Ferner kann eine vorhandene freie Aminogruppe mit einer Mineralsäure oder organischen Säure zu einem Salz umgesetzt werden. Auch die anderen Verbindungen der Formel I können in freier Form oder als solche Salze angewandt werden.

Die nachfolgenden Beispiele sollen die erfindungsgemäßen Verfahrensweisen erläutern, ohne die Erfindung auf die hier stellvertretend genannten Substanzen zu beschränken.

Folgende Abkürzungen werden gebraucht:

| | |
|---|---|
| 1,2,3,4-Tetrahydroisochinolin-3-carbonsäure | Tic |
| Dekahydroisochinolin-3-carbonsäure | Dic |
| Indolin-2-carbonsäure | Idc |
| Oktahydroindol-2-carbonsäure | Oic |
| Benzyloxycarbonyl | Z |
| tert.-Butyloxycarbonyl | Boc |
| tert.-Butyl | tBut, $Bu^1$, tert-$C_4H_9$ |
| 4-Nitrobenzyl | Nb |
| Dicyclohexylcarbodiimid | DCC |
| Dimethylformamid | DMF |
| Dimethylacetamid | DMA |
| N-Ethylmorpholin | NEM |
| Cyclohexylamin | CA |
| Dicyclohexylamin | DCA |

Soweit kein anderes Verfahren angegeben ist, werden die in den folgenden Beispielen beschriebenen Verbindungen zur Analyse und biologischen Bestimmung einer HPLC-Reinigung unterworfen.

Referenzbeispiel 1

N-(1-Carboxy-3-phenylpropyl)-L-alanyl-1,2,3,4-tetrahydroisochinolin-3-carbonsäure

a) 2-Carbobenzoxy-3-carboxy-1,2,3,4-tetrahydroisochinolin (Z-Tic)

188 g (1,05 mol) 3-Carboxy-1,2,3,4-tetrahydroisochinolin gibt man bei 0 °C zu 1050 ml 1 N-NaOH, und tropft dann bei dieser Temperatur unter Rühren gleichzeitig 160 ml Chlorkohlensäurebenzylester und weitere 1050 ml 1N-NaOH ein. Anschließend rührt man 2 Stunden bei Raumtemperatur. Man extrahiert dreimal mit Äther und säuert die alkalisch-wässrige Phase mit konz. HCl auf pH 1 an.Das ausgefallene Öl wird in Essigester extrahiert. Man wäscht die Essigesterlösung mit Wasser, bis die Wasserphase ein pH von 3,0 zeigt. Nach Trocknung über Natriumsulfat kristallisiert das Produkt aus der Essigesterlösung nach Einengen und Anreiben. Man setzt 1,5 Liter Diisopropyläther zu der Kristallsuspension und rührt eine Stunde bei Raumtemperatur, saugt ab, und trocknet das Produkt im Hochvakuum über Phosphorpentoxyd.
Ausbeute: 256,7 g Schmp. 138-39 °C

b) 2-Carbobenzoxy-3-carboxy-1,2,3,4-tetrahydroisochinolin-tert-btuylester

Zur Lösung von 248,8 g (0,8 Mol) 2-Carbobenzoxy-3-carboxy-1,2,3,4-tetrahydroisochinolin in 1600 ml Methylenchlorid gibt man 312 ml tert.Butanol und 8 g 4-Dimethylaminopyridin. Man kühlt auf -5 °C und setzt dann portionsweise die Lösung von 176 g Dicyclohexylcarbodiimid in 350 ml Methylenchlorid zu. Man rührt 5 Stunden bei 0 °C und läßt dann bei Raumtemperatur stehen. Nach Absaugen vom Dicyclohexylharnstoff extrahiert man die Reaktionslösung dreimal mit gesättigter Natriumbicarbonatlösung. Man trocknet über Magnesiumsulfat, engt i.V. bei Raumtemperatur bis zur öligen Konsistenz ein. Es verbleiben 286 g Produkt als gebliches Öl (97 % d.Th.).
NMR: 7.30s (5H); 7.20s (4H); 5,1-4,3m (3H); 5,0s (2H); 1,46s (9H)

(c) 3-Carboxy-1,2,3,4-tetrahydroisochinolin-tert.-butylester-hydrochlorid

284 g 2-Carbobenzoxy-3-carboxy-1,2,3,4-tetrahydroisochinolin-tert-butylester (0.775 Mol) löst man in 3 Liter Methanol, gibt 15 g 10 % Pd-Bariumsulfatkatalysator zu und hydriert mit Wasserstoff bei Normaldruck. Der pH-Wert der Lösung wird durch Zutropfen von 1 n methanolischer HCl auf pH 4,0 (Glaselektrode) gehalten. Man saugt vom Katalysator ab und dampft die Lösung im Vakuum bei Raumtemperatur zur Trockne ein. Das kristallisierte Produkt wird mit wasserfreiem Äther verrieben, abgesaugt und i.V. über Phospohorpentoxyd getrocknet.
Ausbeute: 156 g Schmp. 180 ° (Zers.)
Das Tosylat kann entweder durch Zugabe von methanolischer Toluolsulfonsäure anstelle von methanolischer Salzsäure während der Hydrierung erhalten werden, oder man löst das Hydrochlorid in Wasser, versetzt mit der berechneten Menge Natriumtosylat und kühlt unter Animpfen und Rühren auf ca. 4 °C ab. Dabei fällt das Tosylat in kristalliner Form aus. Die Kristalle werden abfiltriert, mit Wasser gewaschen und getrocknet.
Schmp.: 139 - 140 °C (Zers.)

d) Carbobenzoxy-L-alanyl-3-carboxyl-1,2,34-tetrahydroisochinolin-tert.-butylester

Man suspendiert 27 g (0,15 Mol) 3-Carboxy-1,2,3,4-tetra-hydroisochinolin-tert.-butylesterhydrochlorid in 200 ml Dimethylformamid, kühlt unter Rühren und Feuchtigkeitsausschluß auf -5 °C, und gibt 19 ml (0,15 Mol) N-Äthylmorpholin sowie die Lösung von 33,4 g Carbobenzoxyalanin, 20 g N-Hydroxybenzotriazol und 33 g Dicyclohexylcarbodiimid in 100 ml Dimethylformamid zu. Man rührt eine Stunde bei 0 °C, läßt über Nacht bei + 4 °C stehen und arbeitet dann nach einstündigem Rühren bei Raumtemperatur auf. Man saugt vom ausgeschiedenen Dicyclohexylharnstoff ab und bringt die Reaktionslösung im Hochvakuum bei Raumtemperatur zur Trockne. Das verbliebene Öl nimmt man 1 l Essig auf und wäscht dreimal mit je 150 ml gesättigter Natriumcarbonatlösung, 5 % Kaliumbisulfatlösung und einmal mit Wasser. Nach Trocknung über wasserfreiem Natriumsulfat wird das Lösungsmittel i.V. bei Raumtemperatur abdestilliert.
Ausbeute: 59 g Oel

e) L-Alanyl-3-carboxy-1,2,3,4-tetrahydroisochinolin-tert.-butylesterhydrochlorid (Ala-Tic-O-But)

Man hydriert 15 g Carbobenzoxy-alanyl-3-carboxy-1,2,3,4-tetrahydroisochinolin-tert.-butylester in 400 ml Methanol mit 8 g 10 % Pd/Bariumsulfatkatalysator. Das scheinbare pH der Lösung (Glaselektrode) wird durch Zugabe von 1 N methanolischer Salzsäure bei 4,0 gehalten. Nach 8 Stunden saugt man vom Katalysator ab und dampft die Lösung i.V. bei Raumtemperatur zur Trockne. Den festen Rückstand digeriert man mit Diisopropyläther und trocknet i.V..
Smp.: 110° (Zerg).

Analog Ala-Tic-OBut werden dargestellt:

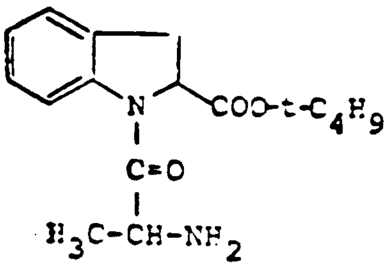

7.3-6.5m (4H); 4,4t (1H); 3,8-3,0 m+d (3H); 1,4s (9H); 1,2d (3H)

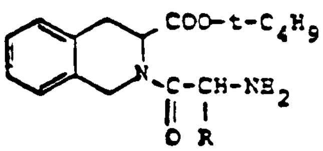

nachstehenden Verbindungen sind folgende Signale gemeinsam:

7,2s (4H); 5,1-4,3m (3H); 3,9-3,0m (4H); 1,4s (9H)

für R : H

7,2s (4H); 3,9-3,0m (5H)

| | |
|---|---|
| 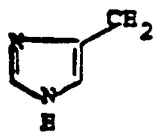 | 13,1s (1H); 7,5s (1H); 6,8s (1H); 2,8m (2H) |
| 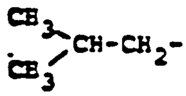 | 1,5m (3H); 0,9d (6H) |
| $CH_2F$- | 5,1-4,3m (5H) |
| 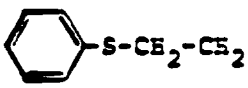 | 7,2-7,0m (9H); 2,7-2,0m (4H) |
| $CH_3$-$CH_2$-CH(-$CH_3$)- | 1,5-1,0m (9H) |
| 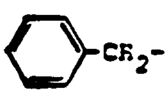 | 7,2s (5H); 2,7d (2H) |
| 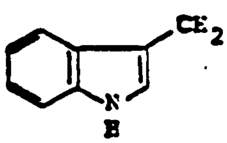 | 7,8-6,4m (9H) |
| HO-$CH_2$- | 3,7d (2H) |

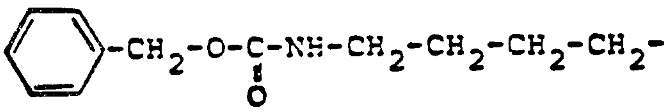

7,1s (5H); 5,0s (2H); 2,4m (2H); 1,8-1,3m (6H)

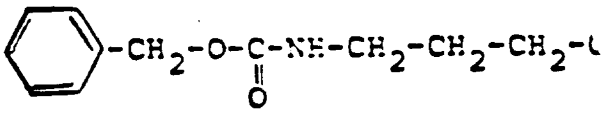

7,1s (5H); 5,0s (2H); 2,4m (2H); 1,8-1,3m (8H)

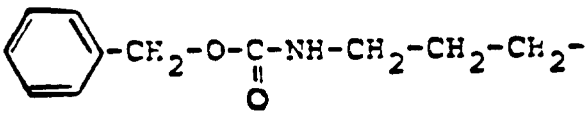

7,1s (5H); 5,0s (2H); 2,4m (2H); 1,8-1,3m (4H)

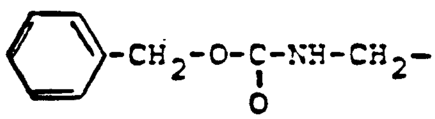

7,1s (5H); 5,0s (2H); 2,3m (2H)

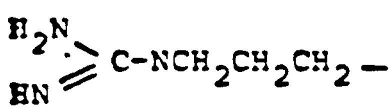

8,3-7,6m (2H); 2,9-1,6m (6H)

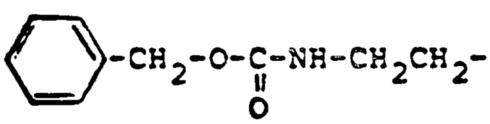

7,1s (5H); 5,0s (2H); 2,4m (2H); 1,7-1,4m (2H)

f) N-(1-Carboxy-3-phenylpropyl)-L-alanyl-1,2,3,4-tetrahydroisochinolin-3-carbonsäure-tert.-butylester

305 mg Alanyl-1,2,3,4-tetrahydroisochinolin-3-carbonsäurebutylester (Ala-Tic-O-But) und 445 mg 4-Phenyl-2-oxo-buttersäure werden in 4 ml Methanol gelöst und mit wässriger 1 N NaOH auf pH 7,5 gebracht. Man fügt 200 mg Natriumcyanoborhydrid hinzu und läßt 36 Std. reagieren. Man engt zur Trockne ein und gewinnt die Substanz durch Säulenchromatographie an Kieselgel (Laufmittelsystem: Chloroform/Methanol/Wasser/Eisessig 20 + 15 + 2 + 1).
Ausbeute: 382 mg; Schmp. ab 120 ° Zers.

g) N-(1-Carboxy-3-phenylpropyl)-L-alanyl-1,2,3,4-tetrahydroisochinolin-3-carbonsäure

350 mg N-(1-Carboxy-3-phenylpropyl)-Ala-Tic-OBut werden in 3 ml wasserfreier Trifluoressigsäure gelöst und 30 Min. bei Raumtemperatur belassen. Man engt i.Vak. ein und verreibt das hinterbleibende Öl mit kaltem Diisopropyläther und Petroläther.
Ausbeute: 276 mg amorphe Substanz
NMR.: 7.20 u. 7.10 (s); 4,3 (s, breit); 3,0-3,9 (m, breit); 1,23 u. 1,15 (d)

Referenzbeispiel 2

N-(1-Carboxy-3-phenyl-3-thiapropyl)-alanyl-1,2,3,4,tetrahydroisochinolin-3-carbonsäure

a) 2-(2-Oxo-propionyl)-1,2,3,4-tetrahydroisochinolin-3-carbonsäuretert.-butylester

In eine auf -50 °C gekühlte Lösung von 700 mg Tic-OBut und 630 mg Dicyclohexylcarbodiimid in 15 ml wasserfreiem Chloroform wird eine gekühlte und frisch destillierte Lösung von 270 mg Brenztraubensäure in 5 ml Chloroform rasch eingerührt. Man beläßt 16 Std. im Tiefkühlschrank (-20 °C) und filtriert den ausgefallenen DC-Harnstoff ab. Die Lösung wird mit $KHSO_4$- und anschließend mit $KHCO_3$-Lösung gewaschen, über $Na_2SO_4$ getrocknet und i.Vak. eingeengt. Die Verbindung (Öl) enthält etwas Dicyclohexylharnstoff: sie wurde zur Analyse über Kieselgel im System $CHCl_3/CH_3OH$ 15:1 chromatographiert. Analog der Kondensation Brenztraubensäure mit Tic-OBut werden folgende Kondensationsprodukte aus Tic, Idc und der entsprechenden $\alpha$-Ketocarbonsäure dargestellt.

| | |
|---|---|
| 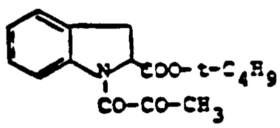 | 7,3-6,5m (4H); 2,4s (3H); 1,4s (9H) |
| 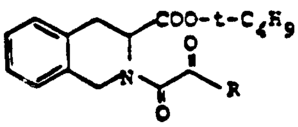 | bei nachstehenden Verbindungen werden einheitlich folgende Signale für das Grundgerüst beobachtet 7,2s (4H); 5,1-4,3m (3H); 3,9-3,0m (2H); 1,4s (9H) |
| 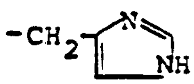 | 13,1s (1H); 7,5s (1H); 6,8s (1H); 3,9-3,0m (4H) |
| $-CH_2-CH(CH_3)_2$ | 2,4-1,0 m+d (9H) |
| 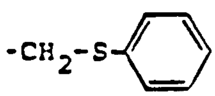 | 7,2-7,0m (9H); 2,5m (2H) |
| $-CH_2F$ | 4,4s (2H) |
| 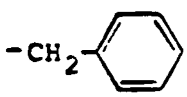 | 7,15s (5H); 3,0s (2H) |
| 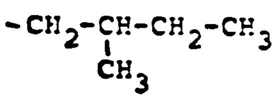 | 2.4-1,9m (3H); 1,5-1,0m (8H) |
| 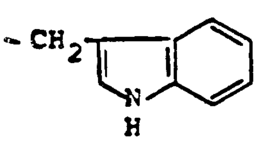 | 7,8-6,4m (9H); 3,9-2,9m (4H) |

b) N-(1-Carboxy-3-phenyl-3-thiapropyl)-alanyl-1,2,3,4-tetrahydroisochinolin-3-carbonsäure-tert.-butylester

1.52 g Pyruvyl-Tic-OBut und 394 mg S-Phenyl-cystein werden in 5 ml Methanol und mit 1 N NaOH (wässrig) auf pH 7.0 gebracht. Man fügt 400 mg Natriumcyanoborhydrid zu und beläßt 24 Stdn. bei Raumtemperatur. Man engt i.Vak. ein, nimmt in Chloroform auf, trocknet über Natriumsulfat und engt ein. Das Produkt wird durch Kieselgelchromatographie (System $CHCl_3/CH_3OH/HAcO/H_2O$ 50/20/5/1) rein erhalten.
Ausbeute: 7,3 - 7,0 m (9H); 5,1 - 4,3 m (3H); 3,9 - 3,0 m (4H); 2,5 m (2H)

c) N-(1-Carboxy-3-phenyl-3-thiapropyl)-alanyl-1,2,3,4-tetrahydroisochinolin-3-carbonsäure

1 g Butylester aus Beispiel 6b wird in 5 ml Trifluoressigsäure gelöst. Man engt nach 30 Min. i.Vak. ein, digeriert den Rückstand mit Diisopropyläther und trocknet über Kaliumhydroxyd.
Ausbeute: 0,95 Trifluoracetat (hygroskopisch)
NMR: 7.4 - 7.1 m (9H); 5,2 - 4,4 m (3H); 3,9 - 3,0 m (4H); 2,5 m (2H); 1,25 m (3H)

Referenzbeispiel 3

N-(1-Carboxy-3-phenylpropyl)-L-alanyl-dekahydroisochinolin-3-carbonsäure

a. L-Dekahydroisochinolin-3-carbonsäure (Dic)
250 g L-1,2,3,4-tetrahydroisochinolin-3-carbonsäure werden in 2 l 90-proz. Essigsäure suspendiert. Man gibt 10 g Rhodium auf Kohle zu und hydriert 24 Stunden bei 60-80 °C und 120 bar. Die filtrierte Lösung wird eingeengt, der Rückstand in 200 ml Essigester aufgenommen und unter kräftigem Rühren in 2 l Diisopropylether eingetropft. Man dekantiert vom harzigen Rückstand, engt die Lösung in Vakuum ein und wiederholt dieselbe Prozedur mit etwa 1/3 der vorher verwendeten Lösungsmittelmenge. Die harzigen Niederschläge werden vereinigt und mit heißem Essigester behandelt, wobei nur ein Teil in Lösung geht. Man läßt unter kräftigem Rühren in 3 l Diethylether-Diisopropylester 1:1 einlaufen, wobei ein flockiger Niederschlag entsteht, der abfiltriert, mit Ether gewaschen und getrocknet wird. Ausbeute: 234 g; dünnschichtchromatographisch nicht einheitlich (Diastereoisomerengemisch). Nach UV-Spektrum und NMR kein Aromat mehr vorhanden, Elementaranalyse korrekt.

Das Material wird ohne weitere Reinigung für die nächste Stufe eingesetzt.

b. N-Benzyloxycarbonyl-L-dekahydroisochinolin-3-carbonsäure (Z-Dic-OH)
58 g L-Dekahydroisochinolin-3-carbonsäure werden in 315 ml 1N NaOH gelöst. Unter kräftigem Rühren läßt man bei 0-5 °C gleichzeitig 48 ml Benzyloxycarbonylchlorid und 370 ml 1N NaOH innerhalb einer Stunde zutropfen. Dabei fällt ein dicker Niederschlag aus. Man rührt nach beendeter Zugabe noch 2 Stdn. weiter, extrahiert dann mit Ether und filtriert den Niederschlag ab ( = Natriumsalz eines Diastereoisomeren "A"; Ausbeute 43 g).
Das Filtrat wird mit konz. HCl auf pH 1,5 - 2 gebracht, wobei sich ein Öl absetzt. Es wird in Essigester aufgenommen. Die Wasserphase wird mit Essigester extrahiert, die vereinigten Essigesterlösungen werden mit Wasser gewaschen, über Natriumsulfat getrocknet und auf ein kleines Volumen (ca. 200 ml) eingeengt. Bei Zugabe von 15-18 ml Cyclohexylamin (Ca) fällt ein Niederschlag aus, der nach Kühlen mit Eiswasser abfiltriert und mit wenig kaltem Essigester gewaschen und dann getrocknet wird. Ausbeute: 48 g, (CA-Salz des Isomerengemisches B).
Das Natriumsalz von "A" wird wie folgt in das CA-Salz übergeführt: Man suspendiert die Verbindung in 500 ml Wasser, überschichtet mit 200 ml Essigester und säuert mit konz. HCl bis pH 1,5 - 2 an. Nach Ausschütteln trennt man die Essigesterphase ab, wäscht mit etwas Wasser und trocknet über Natriumsulfat. Man engt wie oben ein und versetzt mit 16 ml Cyclohexylamin. Das CA-Salz wird wie oben isoliert.
Zur Reinigung wird das CA-Salz von "A" aus der achtfachen Menge Essigester umkristallisiert. Schmelzpunkt: 197 - 198 °C, $[\alpha]_D$ = 7,5 ° (c = 1, in Methanol). Elementaranalyse korrekt.

Das CA-Salz von "B" wird aus der vierfachen Menge Essigester umkristallisiert. Schmp.: 190 ° (sintert ab 187 ° ) 1 $[\alpha]_D$ = + 14,6 ° (c = 1, in Methanol) Elementaranalyse korrekt.

Die freien Säuren werden in bekannter Weise durch Suspendieren der CA-Salze in Wasser/Essigester und Ansäuern mit Citronensäure erhalten. Die Essigesterphasen werden mit wenig Wasser gewaschen und eingeengt. Man erhält Z-Dic-OH als öligen Rückstand.

c. Benzyloxycarbonyl-L-dekahydroisochinolin-3-carbonsäure-tert.-butylester (Z-Dic-$OBu^t$).

41 g Z-Dic-OH ("A") werden in 350 ml Methylenchlorid gelöst. Man gibt 52 ml t-Butanol und 1,3 g 4-Dimethylaminopyridin zu, kühlt auf O °C und tropft unter Rühren die Lösung von 29 g Dicyclohexylcarbodiimid (DCC) in 60 ml Methylenchlorid zu. Man rührt anschließend noch 20 min. bei O °C und 5 h bei Raumtemperatur und filtriert den ausgefallenen Dicyclohexylharnstoff ab. Das Filtrat wird im Vakuum eingedampft, der Rückstand in Essigester aufgenommen, die Lösung nacheinander mit $KHSO_4/K_2SO_4$-Lösung, Natriumhydrogencarbonat (nicht-umgesetztes Na-Salz von Z-Dic-OH fällt aus und wird abfiltriert) und Wasser gewaschen, getrocknet und im Vakuum zur Trockene gebracht. Öliger Rückstand. Ausbeute: 38,5 g $[\alpha]_D$ = -17,3 ° (c = 1, in Methanol). Zur Gewinnung der analogen Verbindung von "B" geht man in derselben Weise vor. Ausbeute 38,1 g, $[\alpha]_D$ = + 6,7 ° (c = 1, in Methanol).

d. L-Dekahydroisochinolin-3-carbonsäure-tert.butylestertosylat (H-Dic-$OBu^t$• TosOH).

27 g der nach Beispiel 1Oc hergestellten Verbindung von "A" werden in 250 ml Methanol unter Zutropfen von 4N TosOH in Methanol an Pd/Kohle bei pH 4,5 (pH-Stat) katalytisch hydriert. Dann engt man die filtrierte Lösung im Vakuum ein, digeriert den kristallinen Rückstand mit Ether und trocknet. Ausbeute: 22,6 g Zur Analyse wird aus Essigester umkristallisiert. Schmp.: 159-160 °C; $[\alpha]_D$ = -6,5 ° (C = 1, in Methanol). Elementaranalyse korrekt. Die Verbindung ist dünnschichtchromatographisch einheitlich.

Analog verfährt man mit der nach Beispiel 1Oc hergestellten Verbindung des Isomerengemisches "B". Dabei wird nach Umkristallisieren aus Essigester eine Substanz vom Schmp. 162-164 °C, $[\alpha]_D$ = + 4,4 ° (c = 1, in Methanol) erhalten. Elementaranalyse korrekt.

e. N-Benzyloxycarbonyl-L-alanyl-L-dekahydroisochinolin-3-carbonsäure-tert.-butylester (Z-Ala-Dic-$OBu^t$)

12,4 g H-Dic-$OBu^t$. TosOH, hergestellt nach Beispiel 1Od mit Verbindung "A", werden in 250 ml Methylenchlorid gelöst. Man gibt 6,7 g Z-Ala-OH und 4,05 g 1-Hydroxybenzotriazol (HOBt) zu, versetzt mit 4,2 ml N-Ethylmorpholin und tropft unter Rühren bei O ° bis 4 ° C die Lösung von 6,6 g Dicyclohexylcarbodiimid (DCC) in 30 ml Methylenchlorid zu. Nach 5 Stunden filtriert man, engt das Filtrat zur Trockene ein, nimmt den Rückstand in Essigester auf und wäscht unterhalb 5 ° C mit 10-prozentiger $KHSO_4$/$K_2SO_4$ (1 : 2), 1M Natriumhydrogencarbonat und Wasser, trocknet über Natriumsulfat und destilliert das Lösungsmittel ab. Harz, leicht löslich in allen organischen Lösungsmitteln. Ausbeute: 15,2 g.

Analog verfährt man mit dem Isomerengemisch "B", das auf dieser Stufe mittels präparativer HPLC an Silicagel im Laufmittel Chloroform/Cyclohexan (9:1) aufgetrennt werden kann.

f. L-Alanyl-L-dekahydroisochinolin-3-carbonsäure-tert.-butylester-tosylat (H-Ala-Dic-$OBu^t$• TosOH)

8,5 g der Z-Verbindung werden analog Beispiel 1Od katalytisch hydriert. Der nach dem Abdestillieren des Lösungsmittels zunächst harzige Rückstand kristallisiert nach einiger Zelt oder beim Verzeihen mit Essigester und wird zur Analyse aus Essigester umkristallisiert. Schmp.: 151-153 ° (Zers.), $[\alpha]_D$ = -28,8 ° (c = 1, in Methanol). Elementaranalyse korrekt.

Analog wird mit dem Isomerengemisch "B" verfahren. Man erhält ein nichtkristallisierendes Harz; korrekte Elementaranalyse.

Analog H-Ala-Dic-$OBu^t$ werden Alanyl-oktahydroindol-2-2-carbonsäure-t-butylester (H-Ala-Oic-$OBu^t$) und die folgenden Verbindungen dargestellt und bevorzugt als Tosylate isoliert (NMR-Daten auf Base bezogen):

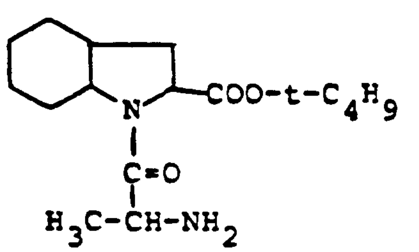

4,9 t (1H); 4,5 -3,0 m (2H); 1,4 s (9H); 1,2 d (3H); 2,0-1,0 m (11H), (H-Ala-Oic-$OBu^t$)

Die Ausgangsaminocarbonsäure Oktahydroindol-2-carbonsäure wird wie folgt hergestellt:

45 g Indol-2-carbonsäure werden in 500 ml 4-proz. Natronlauge gelöst. Man setzt 20 g Raney-Nickel zu und hydriert 24 Stunden bei 40 ° C. Man filtriert, säuert mit konz. HCl an, saugt von Ungelöstem ab und extrahiert die wässrige Phase mehrfach mit Butanol. Die organische Phase wird eingeengt und chromatographiert.

($CHCl_3$/Methanol/Eisessig 5O:2O:5).

Ausbeute: 22 g

Schmp.: 260 ° C.

Analog werden folgende Verbindungen hergestellt. Die Verbindungen zeigen die folgenden NMR-Signale:

| | |
|---|---|
| 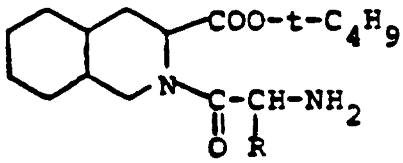 | 5,1-4,3 m (2H); 3,9-3,0 m (2H); 1,4 s (9H); 2,0-1,0m (12H) |
| für R : H | 3,9-3,0 m (4H) |
| 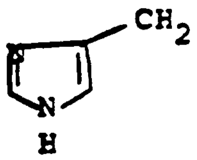 | 13,1 s(1H); 7,5 s (1H); 6,8 s (1H); 2,8 m (2H) |

| | |
|---|---|
| 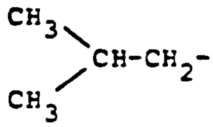 | 1,0-2,0 m (15H); 0,9 d (6H) |
| $-CH_2F$ | 5,1-4,3 m (4H) |
| 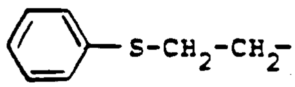 | 7,2 s (5H); 2,7-2,0 m (4H) |
| 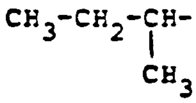 | 1,0-2,0 m (21H) |
| 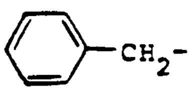 | 7,1 s (5H); 2,7 d (2H) |
| 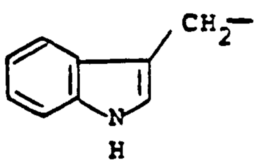 | 7,8-6,4 m (5H); 2,8 m (2H) |
| $HO-CH_2-$ | 3,7 d (2H) |
| 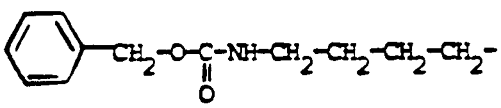 | 7,1 s (5H); 5,0 s (2H); 2,4 m (2H); 2,0-1,0 m (18H) |
| 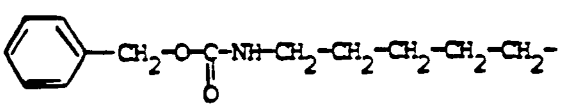 | 7,1 s (5H); 5,0 s (2H); 2,4 m (2H); 2,0-1,0 m (20H) |
| 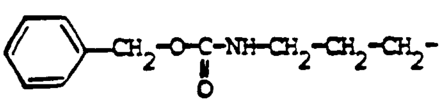 | 7,1 s(5H); 5,0s (2H); 2,4 m (2H); 2,0-1,0 m (16H) |

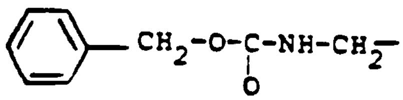

7,1 s (5H); 5,0 s (2H); 2,3 m (2H)

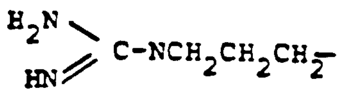

8,3-7,6 m (2H); 2,9-1,0 m (18H)

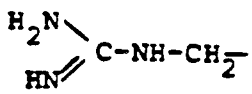

8,3-7,6 m (2H); 2,9-2,5 m (2H)

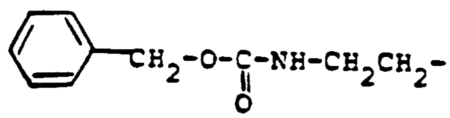

7,1 s (5H); 5,0 s (2H); 2,4 m (2H); 2,0-1,0 m(14H)

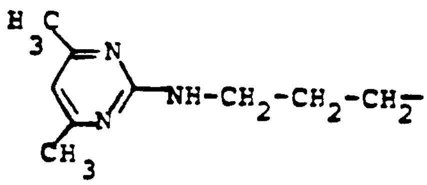

7,4 s(1H); 2,5 m (2H); 2,3 s (6H); 2,0-1,0 m (16H)

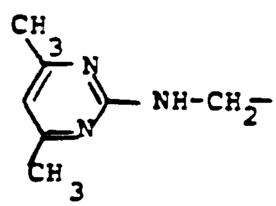

7,4 s(1H); 2,5 m (2H); 2,3 s (6H)

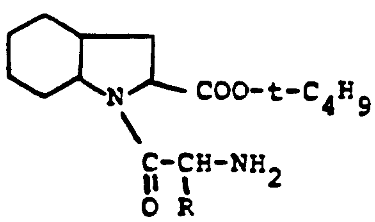

4,9 m (1H); 4,5-3,0 m (2H); 1,4 s (9H); 2,0-1,0 m (11H)

für R : H — 3,9-3,0 m (4H)

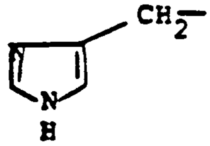

13,1 s (1H); 7,5 s (1H); 6,8 s (1H); 2,8 m (2H)

| | |
|---|---|
| 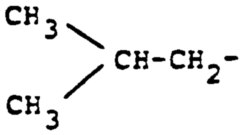 | 1,0-2,0 m (14H); 0,9 d (6H) |
| $-CH_2F$ | 5,1-4,3 m (4H) |
| 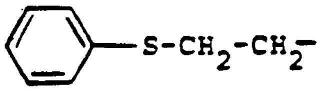 | 7,2-7,0 m (5H); 2,7-2,0 m(4H) |
| 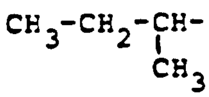 | 1,0-2,0 m (20H) |
| 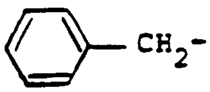 | 7,1 s (5H); 2,7 d (2H) |
| 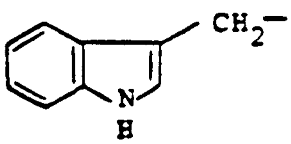 | 7,8-6,4 m (5H); 2,8 m (2H) |
| $HO-CH_2-$ | 3,7 d (2H) |
| 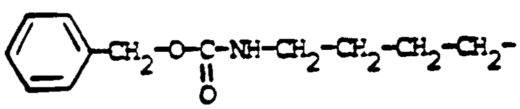 | 7,1 s (5H); 5,0 s (2H); 2,4 m (2H); 2,0-1,0 m (17H) |
| 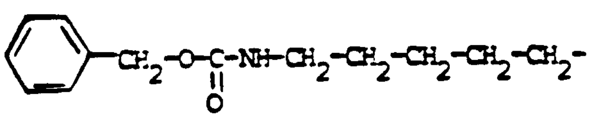 | 7,1 s (5H); 5,0 s (2H); 2,4 m (2H); 2,0-1,0 m (19H) |
| 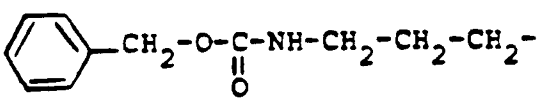 | 7,1 s (5H); 5,0 s (2H); 2,4 m (2H); 2,0-1,0 m (15H) |

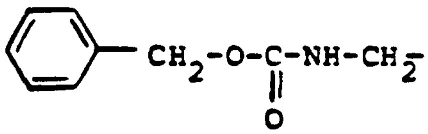

7,1 s (5H); 5,0 s (2H); 2,3 m (2H)

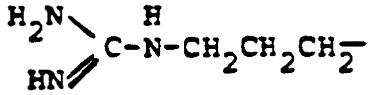

8,3-7,6 m (2H); 2,9-1,0 m (17H)

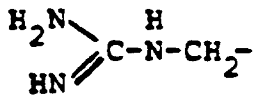

8,3-7,6 m (2H); 2,9-2,5 m (2H)

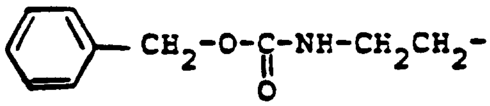

7,1 s (5H); 5,0 s (2H); 2,4 m (2H); 2,0-1,0 m (13H)

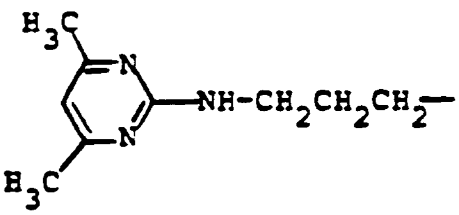

7,4 s(1H); 2,5 m(2H); 2,3 s (6H); 2,0-1,0 m (15H)

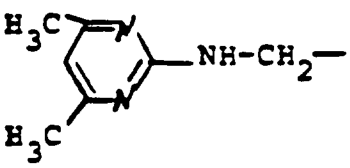

7,4 s(1H); 2,5 m (2H); 2,3 s (6H)

g. N-(1-Carboxy-3-phenylpropyl)-L-alanyl-L-dekahydroisochinolin-3-carbonsäure-tert.-butylester

305 mg Alanyl-dekahydroisochinolin-3-carbonsäure-t-butyl ester (Ala-Dic-OBut) und 445 mg 4-Phenyl-2-oxo-buttersäure werden in 4 ml Methanol gelöst und mit wässriger 1n NaOH auf pH 7,5 gebracht. Man fügt 200 mg Natriumcyanoborhydrid hinzu und läßt 36 Stunden reagieren. Man engt zur Trockne ein und gewinnt die Subatanz durch Säulenchromatographie an Kieselgel (Laufmittelsystem: Chloroform/Methanol/Wasser/Eisessig 20 + 15 + 2 + 1).

Ausbeute: 376 mg; Schmp.: ab 123° Zers.

h. N-(1-Carboxy-3-phenylpropyl)-L-alanyl-L-dekahydroisochinolin-3-carbonsäure

350 mg N-(1-Carboxy-3-phenylpropyl)-Ala-Dic-OBut werden in 3 ml wasserfreier Trifluoressigsäure gelöst und 30 Minuten bei Raumtemperatur belassen. Man engt im Vakuum ein und verreibt das hinterbleibende Öl mit kaltem Diisopropyläther und Petroläther.

Ausbeute: 226 mg amorphe Substanz

NMR: 7,10 (s); 3,0-3,9 (m, breit); 1,23 und 1,15 (d); 2,0-1,0 (m, breit)

Referenzbeispiel 4

L-N-(1-Carbethoxy-3-phenylpropyl)-L-alanyl-L-oktahydroindolin-2-carbonsäure

a) 55 g 2-Brom-4-phenyl-n-buttersäureäthylester, 14,5 g L-Alanin-t-butylester und 45 ml Triethylamin werden 24 h in Tetrahydrofuran aufbewahrt. Man destilliert das Lösungsmittel im Vakuum ab, verteilt den Rückstand zwischen Wasser und Essigester, trennt die Essigesterschicht ab, trocknet über Natriumsulfat und destilliert das Lösungsmittel im Vakuum ab. Der Rückstand wird in Cyclohexan-Essigester (4:1) über Silicagel chromatographiert, wobei neben der Reinigung auch Diastereoisomerentrennung eintritt. Nach

Einengen der entsprechenden Fraktionen wird der t-Butylester durch 20 min. Aufbewahren der Verbindung in Trifluoressigsäure abgespalten. Man destilliert die Trifluoressigsäure im Vakuum ab und destilliert mit Toluol im Vakuum nach.

b) Nun setzt man analog Beispiel 19 b in Dimethylformamid mit 11 g Oktahydroindol-2-carbonsäure-benzylestertosylat, 3,2 ml N-Ethylmorpholin, 3,3 g 1-Hydroxybenzotriazol und 5,5 g Dicyclohexylcarbodiimid um, arbeitet wie dort beschrieben auf und trennt die Diastereomeren durch Chromatographie an Silicagel im System Cyclohexan:Essigester (4:1) auf. Von der chromatographisch einheitlichen Verbindung wird der Benzylester durch katalytische Hydrierung abgespalten.

Referenzbeispiel 5

N-(1-S-Carbethoxy -3-phenyl-propyl)-S-alanyl-cisoctahydroindol-2-S-carbonsäure

a) N-(1-S-Carbethoxy -3-phenyl-propyl)-alanin-benzylester

Durch Extraktion mit Essigester wird aus einer wässr. Sodalösung von 70 g Alanin-benzylester-Toluolsulfonat die freie Base gewonnen. Man versetzt die über wasserfreiem Natriumsulfat getrocknete organische Phase mit 200 ml Dimethylacetamid (DMA) und engt bei RT im Wasserstrahlvakuum ein. Nach Zugabe von 90 g-$\alpha$-Brom-phenyl-buttersäureethylester und 40 ml N-Äthylmorpholin wird die Lösung 4 Tage bei RT belassen. Die Reaktion wird dünnschichtchromatographisch auf Kieselgelplatten (System: Cyclohexan/Essigester 2:1) verfolgt. Man engt im Vakuum bis fast zur Trockne ein, stellt mit 2n methanolischer Salzsäure auf pH 3 und setzt 10 ml Wasser zu. Man extrahiert überschüssigen Bromester und lipophile Produkte in Petroläther, engt die methanolische Phase ein, fügt 3 % Sodalösung (250 ml) zu und extrahiert die Reaktionsprodukte in ein Essigester/Äther-Gemisch (1:1). Nach Trocknung über festem Natriumsulfat wird eingeengt und mit Hilfe einer Kieselgelchromatographie (System Cyclohexan/Essigester 4:1) eine Reinigung und Diastereomerentrennung vorgenommen. Die S,S-Verbindung weist einen niedrigeren $R_f$-Wert auf und fällt in überwiegender Menge an (48%). Die ölige Substanz wird sofort weiter verarbeitet.

b) N-(1-S-Carbethoxy -3-phenyl-propyl)-S-alanin

37 g des Benzylesters werden in 250 ml Ethanol aufgenommen und mit 1 g Pd/Kohle (10 % Pd) bei Normaldruck in 2 Stunden hydrogenolytisch entbenzyliert. Man filtriert den Katalysator ab, engt im Vakuum fast zur Trockene ein und fällt durch Zusatz von Petroläther.

Ausbeute: 33 g; amorph;

NMR: 1,18 (d, 3H); 1,18 (t, 3H); 1,5-2,1 (m, 2H); 2,4-2,8 (m, 2H); 3,0-3,4 (2 m, je 1 H); 4,07 (q, 2H); 4,3-5,5 (b, 2H); 7,17 (s, 5H). DMSO-$d_6$

c) N-(1-S- Carbethoxy -3-phenyl-propyl)-S-alanyl-cisoctahydroindol-2-R,S-carbonsäurebenzylester

5 g N-(1-S- Carbethoxy -3-phenyl-propyl)-S-alanin werden in 20 ml DMF gelöst. Man fügt 2,45 g 1-Hydroxybenzotriazol, 5,1 g cis-Octahydroindol-R,S-carbonsäurebenzylester-hydrochlorid, 2,5 ml N-Ethylmorpholin sowie 4 g Dicyclohexylcarbodiimid zu und rührt 3 Stunden bei Raumtemperatur. Man verdünnt mit 30 ml Essigester und saugt vom Harnstoff ab. Nach Einengen im Vakuum wird in 100 ml Äther aufgenommen und mit wässr. Bicarbonatlösung 2 x extrahiert. Die über Natriumsulfat getrocknete und zur Trockene eingeengte organische Phase wird an Kieselgel chromatographiert. Man eluiert mit Essigester/Petroläther 4:3 und erhält 2 Fraktionen, aus denen durch Einengen im Vakuum farblose Öle erhalten werden.

| Elementaranalyse: | | | | |
|---|---|---|---|---|
| | C | H | N | |
| ber. für $C_{31}H_{40}N_2O_5$ | 71.5 | 7.7 | 5.4 | Ausb. |
| Frakt. 1 | 71.3 | 7.8 | 5.3 | 3.3 g |
| Frakt. 2 | 71.6 | 7.6 | 5.6 | 3.9 g |

d) N-(1-S-Carbethoxy -3-phenyl-propyl)-S-alanyl-cisoctahydroindol-2-S-carbonsäure

3 g des Benzylesters (Fraktion 2) werden in Ethanol gelöst und mit 200 mg 10 % Pd/Kohle bei Normaldruck hydrogenolytisch entbenzyliert. Nach beendeter Wasserstoffaufnahme (etwa 1 Stunde) wird vom Katalsator abfiltriert und die Lösung im Vakuum eingeengt. Man versetzt mit etwas Pentan, erwärmt auf etwa 45 °C und legt ein kräftiges Vakuum an. Es bildet sich ein amorpher, fester Schaum. Ausb.: 2,4 g.

NMR ($CDCl_3$): 1,20 (d, 3H); 1,23 (t, 3H); über Multiplett 1,0-2,9 (11H); 3,0-4,5 (m, 3H); 4,12 (q, 2H); 4,65 (b, 2H); 7,13 (s, 5H).

Referenzbeispiel 6

N-(1-Carbethoxy -3-phenyl-propyl)-alanyl-octahydroindol-2-carbonsäurebenzylester

a) N-tert.Butyloxycarbonyl-alanyl-, octahydroindol-2-carbonsäure-benzylester (Boc-Ala-Oic-OBzl)
19 g Boc-Ala-OH werden in 100 ml DMF gelöst und mit 13 ml N-Ethylmorpholin, 13,5 g HOBt sowie 29,6 g Octahydroindol-2-carbonsäurebenzylester-hydrochlorid versetzt. Man kühlt im Eisbad, fügt 21 g Dicyclohexylcarbodiimid zu und läßt 15 Stunden bei Raumtemperatur rühren. Der ausgefallene Harnstoff wird abgesaugt, das Filtrat im Vakuum eingeengt und in Essigester aufgenommen. Man extrahiert je 3 x mit wässriger $KHSO_4$-, $KHCO_3$- und gesättigter NaCl-Lösung und engt die organische Phase im Vakuum ein. Ausbeute: 38.5 g.
NMR: 1,26 (d, 3H); 1,40 (s, 9H); 1,1-2,4 (m, 12H); 3,2-3,9 (m, 2H); 5,28 (s, 2H); 7,31 (s, 5H).
b) Alanyl-octahydroindol-2-carbonsäurebenzylester-trifluoracetat
21,5 g Boc-Ala-Oic-OBzl werden in 50 ml Trifluoressigsäure gelöst. Man engt im Vakuum ein, digeriert den Rückstand mehrfach mit Diisopropyläther und trocknet im Vakuum. Ausbeute: 21 g. Im NMR fehlt das Protonensignal für die tert. Butylgruppe vollständig.
c) N-(1-Carbethoxy -3-phenyl-propyl)-alanyl-octahydroindol-2-carbonsäurebenzylester
11,1 g Ala-Oic-OBzl.TFA, 7 g $\alpha$-Brom-phenylbuttersäureethylester und 3,3 ml N-Ethylmorpholin werden in 20 ml Dimethylacetamid 4 Tage bei Raumtemperatur gerührt. Man engt im Vakuum weitgehend ein, löst den Rückstand in Methanol, stellt mit wässriger 2n HCl auf pH 2 und extrahiert lipophile Verbindungen mit Petroläther. Die methanolische Phase wird eingeengt, mit 5 % Sodalösung versetzt und das Reaktionsprodukt in Essigester extrahiert. Chromatographie über Kieselgel (System Essigester/Petroläther 5:3) liefert die Titelverbindung als farbloses Öl.
Die nachstehend aufgeführten Beispiele sind nach einer der folgenden Methoden A - H hergestellt, wobei die bezeichneten Methoden wie folgt charakterisiert werden:

Methode A:

Reduktive Aminierung eines Kondensationsproduktes aus Tic oder Dic-$OtC_4H_9$-Ester oder Benzylester bzw. Oic-$OtC_4H_9$-Ester oder Benzylester und einer entsprechenden $\alpha$-Ketocarbonsäure, dargestellt nach Referenzbeispiel 2 mit einem entsprechenden Aminosäurederivat, Abspaltung der Schutzgruppen und gegebenenfalls Verseifung.

Methode B:

Reduktive Aminierung eines Dipeptid-t-Butylesters, dargestellt nach Referenzbeispiel 1 mit einem entsprechenden $\alpha$-Ketocarbonsäurederivat und ggfs. Abspaltung der Schutzgruppen nach Referenzbeispiel 3

Methode C:

Reduktive Aminierung nach Methode A und mit einer $\omega$-geschützten bifunktionellen Aminosäure, Abspaltung einer Schutzgruppe aus dem eingesetzten Aminosäurederivat.

Methode D:

Oxidation der zugrunde liegenden Schwefel-Verbindung zum Sulfoxid

Methode E:

Oxidation der zugrunde liegenden Schwefel-Verbindung zum Sulfon

Methode F:

Kondensation einer entsprechenden N-alkylierten α-Aminosäure mit einem Aminosäureester (z.B. Dic-$OBu^t$ oder Dic-OBzl bzw. Oic-$Bu^t$ oder Oic-OBzl) und Abspaltung der Schutzgruppe
Falls nicht anders angegeben, gelten für die nachstehenden Verbindungen folgende NMR-Daten (θ - Werte in ppm; TMS als Standard):

| A = Benzolring: | |
|---|---|
| für n = 1 | 7,3s 84H); 5,1-4,3m (3H) 3,9-3.0m, (4H) |
| n = 0 | 7.2-6.5m (4H); 4,9 (1H) 3,6 - 3,0m (4H) |

| A = Cyclohexanring | |
|---|---|
| für n = 1 | 5,1 - 4,3m (2H); 3,9-3,0m (3H); 2.0-1.0m (12H) |
| n = 0 | 4,9m (1H); 4,5-3,0m (3H); 2.0-1.0m (11H) |

Im Falle der Ethylester treten noch folgende Signale auf:
4,2 q 7 Hz (2H)
1,2 t 7 Hz (3H)

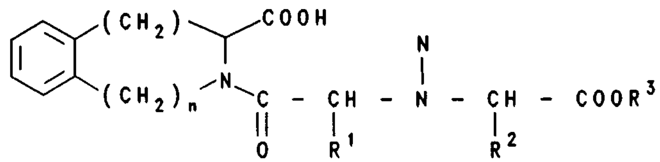

| | n | $R^1$ | $R^2$ | $R^3$ | Methode | NMR |
|---|---|---|---|---|---|---|
| 1 | 0 | $CH_3$ | 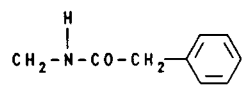 | $C_2H_5$ | A | 7,2-6,5m (9H); 3,8-3,0m (6H); 2,9m (2H); 1,2d (3H) |
| 2 | 0 | $CH_3$ | 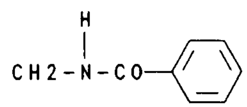 | $C_2H_5$ | A | 7,8-6,5m (9H); 2,9m (2H); 1,2d (3H) |

| | n | $R^1$ | $R^2$ | $R^3$ | Methode | NMR |
|---|---|---|---|---|---|---|
| 3 | 0 | $CH_3$ | 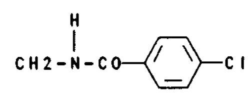 | $C_2H_5$ | A | 7,8-6,5m (8H); 2,9m (2H); 1,2d (3H) |
| 4 | 0 | $CH_3$ | 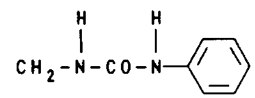 | $C_2H_5$ | A | 7,4-6,5m (9H); 2,9m (2H); 1,2d (3H) |
| 5 | 0 | $CH_3$ | 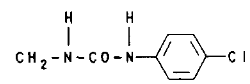 | $C_2H_5$ | A | 7,7-6,5m (8H); 2,9m (2H); 1,2d (3H) |
| 6 | 0 | $CH_3$ | 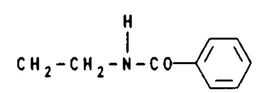 | $C_2H_5$ | A | 7,6-6,5m (9H); 2,9m (2H); 1,5m (2H); 1,2d (3H) |

| | n | $R^1$ | $R^2$ | $R^3$ | Methode | NMR |
|---|---|---|---|---|---|---|
| 7 | 0 | $CH_3$ | 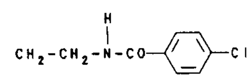 | $C_2H_5$ | A | 7,6-6,5m (8H); 2,9m (2H); 1,5m (2H); 1,2d (3H) |
| 8 | 0 | $CH_3$ | 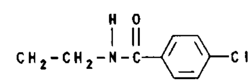 | H | A | 7,6-6,5m (8H), 2,9m (2H); 1,5m (2H); 1,2d (3H) |
| 9 | 0 | $CH_3$ | $CH_2$-$CH_2$-$CH_2$-$N(C_2H_5)_2$ | $C_2H_5$ | A | 2,6-2,4m (6H); 1,5m (4H); 1,2d (3H); 1,0t (6H) |
| 10 | 0 | $CH_3$ | 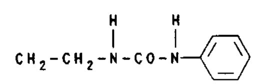 | $C_2H_5$ | A | 7,4-6,5m (9H); 2,9m (2H); 1,5m (2H); 1,2d (3H) |

| | n | $R^1$ | $R^2$ | $R^3$ | Methode | NMR |
|---|---|---|---|---|---|---|
| 11 | 0 | $CH_3$ | 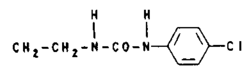 | $C_2H_5$ | A | 7,5-6,5m (8H); 2,9m (2H); 1,5m (2H); 1,2d (3H) |
| 12 | 0 | $CH_3$ | 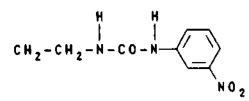 | $C_2H_5$ | A | 7,8-6,5m (8H); 2,9m (2H); 1,5m (2H); 1,2d (3H) |
| 13 | 0 | $CH_3$ | H H<br>\| \|<br>$CH_2$-$CH_2$-N-CO-N-$CH_3$ | $C_2H_5$ | A | 3,0-2,6m + n (5H); 1,5m (2H); 1,2d (3H) |
| 14 | 0 | $CH_3$ | 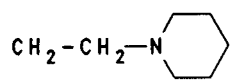 | $C_2H_5$ | A | 2,6-2,3m (6H); 1,8-1,2m (8F); 1,2d (3H) |

| | n | $R^1$ | $R^2$ | $R^3$ | Methode | NMR |
|---|---|---|---|---|---|---|
| 15 | 0 | $CH_3$ | 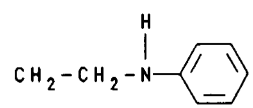 | $C_2H_5$ | A | 7,2-6,5m (9H); 2,4m (2H); 1,5 (2H); 1,2d (3H) |
| 16 | 0 | $CH_3$ | 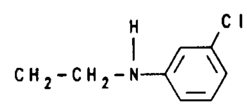 | $C_2H_5$ | A | 7,6-6,5m (8H); 2,4m (2H); 1,5m (2H); 1,2d (3H) |

| | n | $R^1$ | $R^2$ | $R^3$ | Methode | NMR |
|---|---|---|---|---|---|---|
| 17 | 0 | $CH_3$ | 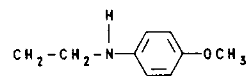 | $C_2H_5$ | A | 7,2-6,4m (8H); 3,9m (3H); 2,4m (2H); 1,5m (2H); 1,2d (3H) |
| 18 | 0 | $CH_3$ | $(CH_2)_5-NH_2$ | H | C | 2,4m (2H); 1,8-1,2m (8H); 1,2d (3H) |
| 19 | 0 | $CH_3$ | $(CH_2)_4-NH_2$ | H | C | 2,4m (2H); 1,8-1,2m (6H); 1,2d (3H) |
| 20 | 0 | $CH_3$ | 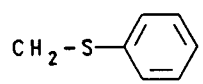 | $C_2H_5$ | A | 7,4-6,5m (9H); 2,5m (2H); 1,2d (3H) |
| 21 | 0 | $CH_3$ | 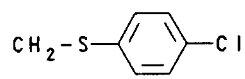 | $C_2H_5$ | A | 7,4-6,4m (8H); 2,5m (2H); 1,2d (3H) |

| | n | $R^1$ | $R^2$ | $R^3$ | Methode | NMR |
|---|---|---|---|---|---|---|
| 22 | 0 | $CH_3$ | 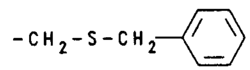 | $C_2H_5$ | A | 7,3-6,5m (9H), 3,6-3,0m+n (6H); 2,4m (2H); 1,2d (3H) |
| 23 | 0 | $CH_3$ | $-CH_2-S-CH-(CH_3)_2$ | $C_2H_5$ | A | 2,7-2,3m (3H); 1,2d (3H); 0,9d (6H) |
| 24 | 0 | $CH_3$ | 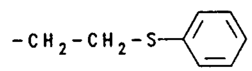 | $C_2H_5$ | A | 7,3-6,5m (9H); 2,7m (2H); 1,5m (2H); 1,2d (3H) |
| 25 | 0 | $CH_3$ | 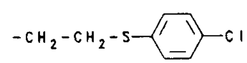 | $C_2H_5$ | A | 7,5-6,5m (8H); 2,7m (2H); 1,5m (2H); 1,2d (3H) |
| 26 | 0 | $CH_3$ | 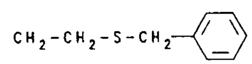 | $C_2H_5$ | A | 3,9-3,0m+s (6H); 2,4m (2H); 1,5m (2H); 1,2d (3H); |

| | n | $R^1$ | $R^2$ | $R^3$ | Methode | NMR |
|---|---|---|---|---|---|---|
| 27 | 0 | $CH_3$ | $CH_2$-$CH_2$-S-$CH(CH_3)_2$ | $C_2H_5$ | A | 2,6-2,3m (3H); 1,5m (2H); 1,2d (3H); 1,0d (6H) |
| 28 | 0 | $CH_3$ | 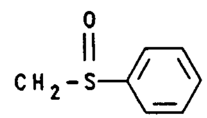 | $C_2H_5$ | D | 7,5-6,5m (9H); 2,6m (2H); 1,2d (3H) |
| 29 | 0 | $CH_3$ | 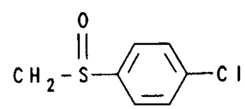 | $C_2H_5$ | D | 7,4-6,5m (8H); 2,6m (2H); 1,2d (3H) |
| 30 | 0 | $CH_3$ | 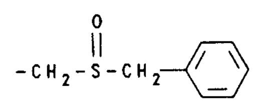 | $C_2H_5$ | D | 7,3-6,5m (9H); 4,1s (2H); 2,6m (2H); 1,2d (3H) |

| | n | $R^1$ | $R^2$ | $R^3$ | Methode | NMR |
|---|---|---|---|---|---|---|
| 31 | 0 | $CH_3$ | O<br>‖<br>$-CH_2-S-CH(CH_3)_2$ | $C_2H_5$ | D | 2,8-2,5m (3H); 1,2d (3H); 1,0d (6H) |
| 32 | 0 | $CH_3$ | 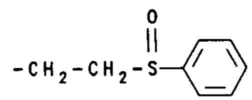 | $C_2H_5$ | D | 7,4-6,5m (9H); 2,6m (2H); 1,5m (2H); 1,2d (3H) |
| 33 | 0 | $CH_3$ | 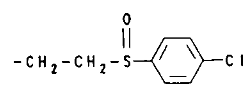 | $C_2H_5$ | D | 7,5-6,9m (8H); 2,6m (2H); 1,5m (2H); 1,2d (3H) |
| 34 | 0 | $CH_3$ | 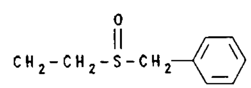 | $C_2H_5$ | D | 7,3-6,5m (9H); 4,1s (2H); 2,7m (2H); 1,5m (2H); 1,2d (3H) |

| | n | $R^1$ | $R^2$ | $R^3$ | Methode | NMR |
|---|---|---|---|---|---|---|
| 35 | 0 | $CH_3$ | O<br>‖<br>$CH_2-CH_2-S-CH(CH_3)_2$ | $C_2H_5$ | D | 2,8-2,5m (3H); 1,5m (2H); 1,2d (3H); 1,1d (6H) |
| 36 | 0 | $CH_3$ | 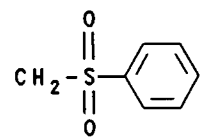 | $C_2H_5$ | E | 7,8-6,5m (9H); 2,8m (2H); 1,2d (3H) |
| 37 | 0 | $CH_3$ | 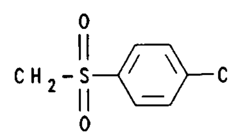 | $C_2H_5$ | E | 7,7-6,5m (8H); 2,8m (2H); 1,2d (3H) |

| | n | $R^1$ | $R^2$ | $R^3$ | Methode | NMR |
|---|---|---|---|---|---|---|
| 38 | 0 | $CH_3$ | 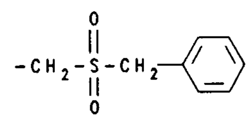 | $C_2H_5$ | E | 7,6-6,5 (9H); 4,3s (2H); 2,8m (2H); 1,2d (3H) |
| 39 | 0 | $CH_3$ | 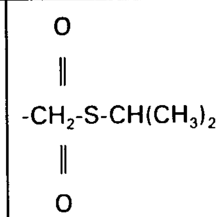 | $C_2H_5$ | E | 3,0-2,7m (3H); 1,2d+t (9H) |
| 40 | 0 | $CH_3$ | 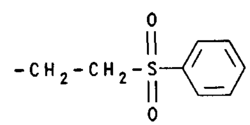 | $C_2H_5$ | E | 7,7-6,5 (9H); 2,8m (2H); 1,5m (2H); 1,2d (3H) |

| | n | $R^1$ | $R^2$ | $R^3$ | Methode | NMR |
|---|---|---|---|---|---|---|
| 41 | 0 | $CH_3$ | 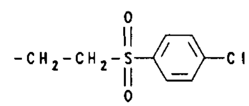 | $C_2H_5$ | E | 8,0-6,5m (8H); 2,8m (2H); 1,5m (2H); 1,2d (3H) |
| 42 | 0 | $CH_3$ | 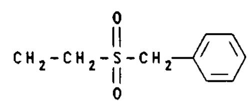 | $C_2H_5$ | E | 7,2-6,5m (9H); 4,2s (2H); 2,8m (2H); 1,5m (2H); 1,2d (3H) |
| 43 | 0 | $CH_3$ | O<br>‖<br>$CH_2$-$CH_2$-S-$CH(CH_3)_2$<br>‖<br>O | $C_2H_5$ | E | 3,0-2,7m (3H); 1,5m (2H); 1,1d+t (9H) |
| 44 | 0 | $CH_3$ | $CH_2$-OH | $C_2H_5$ | A | 3,6-3,0m (6H); 1,2d (3H) |

| | n | $R^1$ | $R^2$ | $R^3$ | Methode | NMR |
|---|---|---|---|---|---|---|
| 45 | 0 | $CH_3$ | 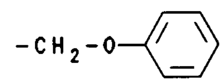 | $C_2H_5$ | A | 7,3-6,5m (9H); 3,9-3,0m (6H); 1,2d (3H) |
| 46 | 0 | $CH_3$ | 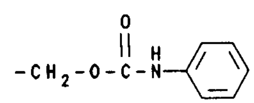 | $C_2H_5$ | A | 7,6-6,5m (9H); 4,0m (2H); 1,2d (3H) |
| 47 | 0 | $CH_3$ | $CH_2$-COOH | H | A | 2,1m (2H); 1,2d (3H) |
| 48 | 0 | $CH_3$ | $CH_2$-$CONH_2$ | H | F | 6,5bs (2H); 2,1m (2H); 1,2d (3H) |
| 49 | 0 | $CH_3$ | $CH_2$-$CON(CH_3)_2$ | H | F | 3,9-3,0m+2s (10H); 2,1m (2H); 1,2d (3H) |

| | n | $R^1$ | $R^2$ | $R^3$ | Methode | NMR |
|---|---|---|---|---|---|---|
| 50 | 0 | $CH_3$ | 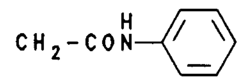 | H | F | 7,3-6,5m (9H); 2,1m (2H); 1,2d (3H) |
| 51 | 0 | $CH_3$ | 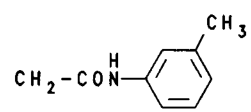 | H | F | 7,3-6,5m (8H); 2,3s (3H); 2,2m (2H); 1,2d (3H) |
| 52 | 0 | $CH_3$ | 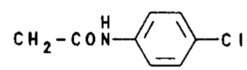 | H | F | 7,7-6,5m (8H); 2,3m (2H); 1,2d (3H) |
| 53 | 0 | $CH_3$ | $CH_2$-$CH_2$-COOH | $C_2H_5$ | A | 2,1m (2H); 1,5m (2H); 1,2d (3H) |
| 54 | 0 | $CH_3$ | $CH_2$-$CH_2$-$CONH_2$ | $C_2H_5$ | A | 2,1m (2H); 1,5m (2H); 1,2d (3H) |
| 55 | 0 | $CH_3$ | $CH_2$-$CH_2$-$CON(CH_3)_2$ | $C_2H_5$ | A | 3,0s (6H); 1,5m (2H); 1,2d (3H) |

| | n | $R^1$ | $R^2$ | $R^3$ | Methode | NMR |
|---|---|---|---|---|---|---|
| 56 | 0 | $CH_3$ | 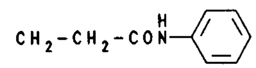 | $C_2H_5$ | A | 7,4-6,5m (9H); 2,1m (2H); 1,5m (2H); 1,2d (3H) |
| 57 | 0 | $CH_3$ | 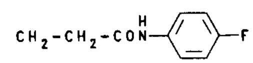 | H | A | 7,3-6,5m (8H); 2,1m (2H); 1,5m (2H); 1,2d (3H) |
| 58 | 0 | $CH_3$ | 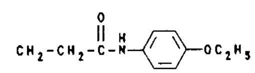 | $C_2H_5$ | A | 7,3-6,4m (8H); 3,6q (3H); 2,1m (2H); 1,5m (2H); 1,2d+t (6H) |
| 59 | 0 | $CH_3$ | H<br>$CH_2$-$CH_2$-CON-CH$(CH_3)_2$ | $C_2H_5$ | A | 2,8-2,2m (3H); 1,5m (2H); 1,2d (3H); 1,0d (6H) |
| 60 | 0 | $CH_3$ | 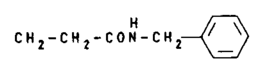 | $C_2H_5$ | A | 7,4-6,5m (9H); 5,1-4,3m+s (5H); 2,1m (2H); 1,5m (2H); 1,2d (3H) |

| | n | $R^1$ | $R^2$ | $R^3$ | Methode | NMR |
|---|---|---|---|---|---|---|
| 61 | 0 | $CH_3$ | 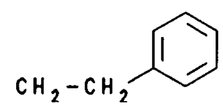 | $C_2H_5$ | B | 7,2-6,5m (9H); 2,7m (2H); 1,7m (2H); 1,2d (3H) |
| 62 | 0 | $CH_3$ | 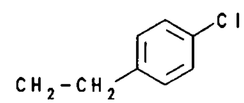 | H | B | 7,5-6,5m (8H); 2,7m (2H); 1,7m (2H); 1,2d (3H) |
| 63 | 0 | $CH_3$ | 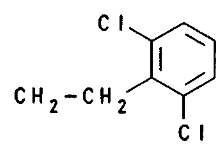 | H | B | 7,6-6,5m (7H); 2,8m (2H); 1,7m (2H); 1,2d (3H) |
| 64 | 0 | $CH_3$ | 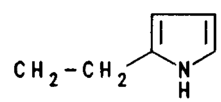 | H | B | 7,3-6,0m (7H); 2,8m (2H); 1,7m (2H); 1,2d (3H) |

| | n | $R^1$ | $R^2$ | $R^3$ | Methode | NMR |
|---|---|---|---|---|---|---|
| 65 | 0 | $CH_3$ | 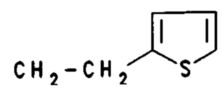 | H | B | 7,3-6,5m (7H); 2,9m (2H); 1,7m (2H); 1,2d (3H) |
| 66 | 0 | $CH_3$ | 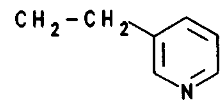 | H | B | 8,6-6,5m (8H); 2,9m (2H); 1,7m (2H); 1,2d (3H) |
| 67 | 0 | $CH_3$ | 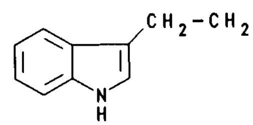 | $C_2H_5$ | B | 7,8-6,5m (10H); 2,9m (2h); 1,7m (2H); 1,2d (3H) |

| | n | $R^1$ | $R^2$ | $R^3$ | Methode | NMR |
|---|---|---|---|---|---|---|
| 68 | 0 | $CH_3$ | 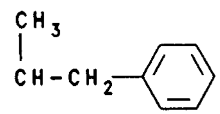 | $C_2H_5$ | B | 7,2-6,5m (9H); 2,9m (2H); 1,8m (1H); 1,2d (3H); 1,1d (3H) |
| 69 | 0 | $CH_3$ | 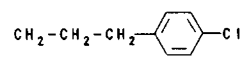 | $C_2H_5$ | B | 7,4-6,5m (8H); 2,7-2,3m (2H); 1,8-1,3m (4H); 1,2d (3H) |
| 70 | 0 | $CH_3$ | 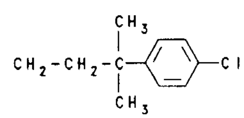 | $C_2H_5$ | B | 7,3-6,3m (8H); 3,9m (3H); 1,9-1,2m (4H); 1,2d (3H); 1,0s (6H) |

| | n | $R^1$ | $R^2$ | $R^3$ | Methode | NMR |
|---|---|---|---|---|---|---|
| 71 | 0 | $CH_3$ | 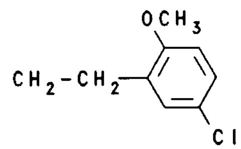 | $C_2H_5$ | B | 7,2-6,3m (7H); 3,9m (3H); 2,7m (2H); 1,8m (2H); 1,2d (3H) |
| 72 | 0 | $CH_3$ | 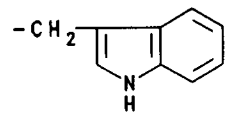 | $C_2H_5$ | B | 7,8-6,5m (10H); 2,9m (2H); 1,2d (3H) |
| 73 | | $CH_3$ | 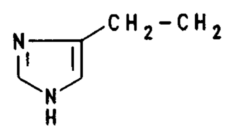 | $C_2H_5$ | B | 7,7-6,5m (6H); 2,9m (2H); 1,7m (2H); 1,2d (3H) |

| | n | $R^1$ | $R^2$ | $R^3$ | Methode | NMR |
|---|---|---|---|---|---|---|
| 74 | 0 | $CH_3$ | 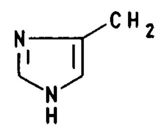 | $C_2H_5$ | B | 7,7-6,5m (6H); 2,9m (2H); 1,2d (3H) |

**Patentansprüche**

**Patentansprüche für folgende Vertragsstaaten : BE, CH, DE, FR, GB, IT, LI, LU, NL, SE**

**1.** Verbindung der Formel 1 b

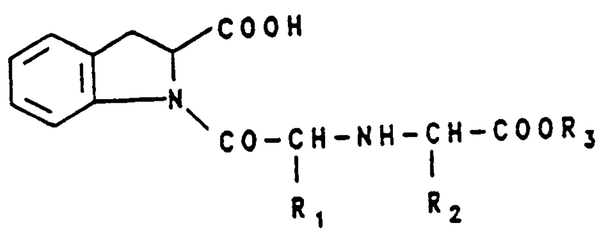

(1b)

in welcher

$R_1$ und $R_2$ gleich oder verschieden sind und

a) Alkyl oder Alkenyl mit bis zu 6 C-Atomen bedeuten, welche jeweils gegebenenfalls substituiert sind durch einen oder mehrere gleiche oder verschiedene Substituenten aus der Reihe

1. ($C_5$-$C_7$)-Cycloalkyl,
2. ($C_5$-$C_7$)-Cycloalkenyl,
3. Hydroxy,
4. Aryloxy, das gegebenenfalls substituiert ist durch Methoxy, Ethoxy, Carboxy, Carbamoyl, Amino, ($C_1$-$C_6$)-Alkylamino oder durch Halogen und/oder Nitro,
5. Amino,
6. Monoalkylamino bis zu 7 C-Atomen, das im Alkylrest gegebenenfalls substituiert ist durch Hydroxy, Carboxy, Carbamoyl, Ethoxycarbonyl, Amino, ($C_1$-$C_6$)-Alkylamino, Di-($C_1$-$C_6$)-alkylamino, Piperidino oder Morpholino,
7. Dialkylamino mit bis zu 7 C-Atomen, das im Alkylrest gegebenenfalls wie unter a) 6 beschrieben substituiert ist,
8. Cycloalkylamino mit bis zu 7 C-Atomen, das im Alkylrest gegebenenfalls wie unter a) 6 beschrieben substituiert ist,
9. Dicycloalkylamino mit bis zu 7 C-Atomen, das im Alkylrest gegebenenfalls wie unter a) 6 beschrieben substituiert ist,
10. ($C_1$-$C_6$)-Alkoxycarbonylamino,
11. Aryloxycarbonylamino, das im Arylteil gegebenenfalls mono- oder disubstituiert ist durch ($C_1$-$C_2$)-Alkoxy, Methylendioxy, Amino, Hydroxy, Acetoxy, Carboxy, Carbamoyl, Ethoxycarbonyl, Halogen oder Nitro,
12. Aralkyloxycarbonylamino, das im Arylteil gegebenenfalls wie unter a) 11 beschrieben mono- oder di-substituiert ist,
13. ($C_1$-$C_6$)-Alkylureido,
14. Arylureido, das im Arylteil gegebenenfalls wie unter a) 11 beschrieben durch gleiche oder verschiedene Reste mono- oder di-substituiert ist,
15. Aralkylureido, das im Arylteil gegebenenfalls wie unter a) 11 beschrieben mono- oder di-substituiert ist,
16. Formyl,
17. Alkanoylamino mit bis zu 10 C-Atomen,
18. Aroylamino mit bis zu 10 C-Atomen, das im Arylteil gegebenenfalls wie unter a) 11 beschrieben mono- oder disubstituiert ist,
19. Aralkanoylamino mit bis zu 10 C-Atomen, das im Arylteil gegebenenfalls wie unter a) 11 beschrieben mono- oder disubstituiert ist,
20. Arylamino, das im Arylteil gegebenenfalls wie unter a) 11 durch gleiche oder verschiedene Reste mono- oder di-substituiert ist,
21. Aralkylamino, das im Arylteil gegebenenfalls wie unter a) 11 beschrieben mono- oder disubstituiert ist,
22. Alkylmercapto, Alkylsulfinyl oder Alkylsulfonyl mit bis zu 7 C-Atomen, das jeweils im Alkylteil gegebenenfalls substituiert ist durch Methoxy, Ethoxy, Hydroxy, Carboxy, Carbamoyl, Ethoxycarbonyl, Amino oder ($C_1$-$C_6$)-Alkylamino,

23. Phenylmercapto, Phenylsulfinyl oder Phenylsulfonyl, das im Arylteil gegebenenfalls wie unter a) 22 beschrieben oder durch Halogen, Nitro oder Sulfamoyl substituiert ist,
24. Benzylmercapto, Benzylsulfinyl oder Benzylsulfonyl, das im Alkylteil gegebenenfalls wie unter a) 22 und das im Arylteil gegebenenfalls wie vorstehender Alkylteil oder wie unter a) 23 beschrieben substituiert ist,
25. Carboxy,
26. Carbamoyl,
27. Alkylcarbamoyl mit bis zu 6 C-Atomen,
28. Cycloalkylcarbamoyl mit bis zu 6 C-Atomen,
29. Dialkylcarbamoyl mit bis zu 6 C-Atomen,
30. Arylcarbamoyl,
31. Aralkylcarbamoyl,
32. Guanidino,
33. Phenyl, Naphthyl, Dihydronaphthyl oder Tetrahydronaphthyl, die jeweils gegebenenfalls mono- oder di-substituiert sind durch Halogen, Hydroxy, Acetoxy, Carboxy, Carbamoyl, Sulfamoyl, Nitro, Methyl, Ethyl, Methoxy und/oder Ethoxy,
34. einen 5- bis 7-gliedrigen monocyclischen oder 9- bis 10-gliedrigen bicyclischen Heterocyclen-Rest, gegebenenfalls 1 bis 2 S- oder O-Atome und/oder bis zu 4 N-Atome pro Ring enthaltend, der gegebenenfalls substituiert durch Halogen, Hydroxy, Carboxy, Carbamoyl, Sulfamoyl, Nitro, Methoxy und/oder Ethoxy;

b) Cycloalkyl oder Cycloalkenyl mit je 5-7 C-Atomen bedeuten;
c) Aryl oder teilhydriertes Aryl mit 6 bis 10 C-Atomen bedeuten;
d) ein mono- oder bicyclischer Heterocyclen-Rest mit 5-7 bzw. 8-10 Gliedern, davon 1-2 -S- oder -O- und/oder bis zu 4 N-Atomen bedeuten, das gegebenenfalls wie unter a) 34 beschrieben substituiert ist und

$R_3$ Wasserstoff, Alkyl mit 1-6 C-Atomen, Alkenyl mit 2-6 C-Atomen, Aralkyl mit 7 - 14 C-Atomen inklusive p-Nitrobenzyl bedeutet,
wobei, falls im einzelnen nicht anders definiert, Aryl 6-10 C-Atome aufweist und gegebenenfalls durch Halogen, $(C_1$-$C_6)$-Alkyl oder $(C_1$-$C_6)$-Alkoxy substituiert ist, und
Aralkyl, Benzyl oder Phenethyl bedeutet und gegebenenfalls durch Halogen,
$(C_1$-$C_6)$-Alkyl oder $(C_1$-$C_6)$-Alkoxy im Phenylkern substituiert ist,
oder deren physiologisch verträglichen Salze.

**2.** Verbindung gemäß Anspruch 1, in welcher $R_1$ Methyl und $R_2$-Phenethyl oder durch Halogen, Methyl oder Methoxy substituiertes Phenethyl ist.

**3.** Verbindung gemäß Anspruch 1 oder 2, in welcher $R_3$ Wasserstoff, Ethyl, Butyl, t-Butyl, Benzyl, p-Nitrobenzyl bedeutet.

**4.** Verbindung gemäß Anspruch 1 oder 3, in welcher $R_1$ die Seitenkette einer natürlich vorkommenden L-Aminosäure oder deren Ethers, Esters oder Amids ist.

**5.** Verbindung gemäß Anspruch 4, in welcher $R_1$ Methyl, Isobutyl, Methylthioethyl, Carboxymethyl, Carboxyethyl, Amino-n-butyl, Guanidino-n-propyl, Imidazol-4-ethyl, Benzyl, 4-Hydroxybenzyl oder 3-Indol-methyl bedeutet.

**6.** Verbindung gemäß Anspruch 1 oder 3, in welcher $R_1$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl, Naphthyl, Di- oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

**7.** Verbindung gemäß Anspruch 1 oder 3, in welcher $R_2$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl, Naphthyl, Di- oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

8. Verbindung gemäß einem der Ansprüche 1-7, in welcher die zwei oder drei chiralen Zentren an $\alpha$-C-Atomen in der L-Konfiguration vorliegen.

9. Verfahren zur Herstellung einer Verbindung gemäß einem der Ansprüche 1-8, dadurch gekennzeichnet, daß man
(a) eine Verbindung der Formel II' mit einer Verbindung der Formel III umsetzt,

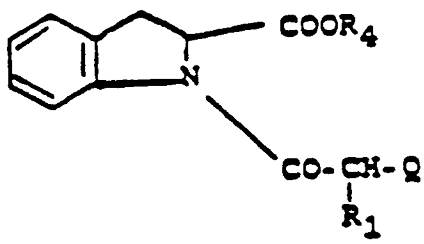

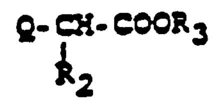

(II') (III)

in denen $R_1$, $R_2$ und $R_3$ wie im Anspruch 1 definiert sind, ein Q eine nucleofuge Gruppe und das andere Q $-NH_2$ bedeutet und $R_4$ für H, Methyl, Ethyl, Benzyl oder tert.-Butyl steht, und gegebenenfalls einen erhaltenen Ester in die Carbonsäure ($R_2$ und/oder $R_4$ = Wasserstoff) überführt oder
(b) eine Verbindung (IV)

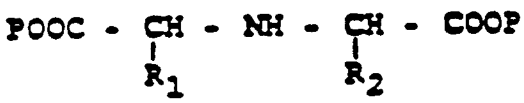

(IV)

worin $R_1$ und $R_2$ wie im Anspruch 1 definiert sind, P H bedeutet, eines der beiden P jedoch auch die anderen Bedeutungen von $R_3$ im Anspruch 1 inklusive tert.-Butyl haben kann, mit einer Verbindung der Formel V'

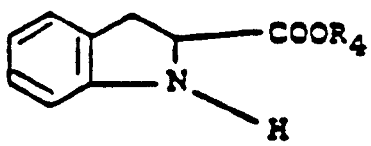

(V')

worin $R_4$ wie oben definiert ist, in Gegenwart eines Kondensationsmittels umsetzt und gegebenenfalls eine oder beide Estergruppen in die Carbonsäure überführt oder
(c) eine Verbindung der Formel VI' mit einer Verbindung der Formel VII umsetzt

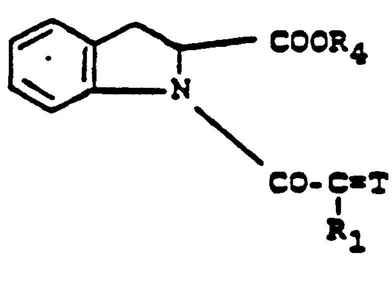

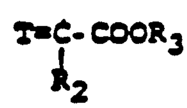

(VI') (VII)

in denen $R_1$, $R_2$ und $R_3$ wie im Anspruch 1 und $R_4$ wie oben definiert sind, ein T für ein Wasserstoffatom und eine $NH_2$-Gruppe und das andere T für ein Sauerstoffatom steht, und die

erhaltene Schiff'sche Base reduziert, in den nach (a)-(c) erhaltenen Verbindungen Schwefelfunktionen gegebenenfalls zum Sulfoxid oder zum Sulfon oxidiert
und die so erhaltene Verbindung der Formel Ib gegebenenfalls in ihr Salz überführt.

**10.** Mittel, enthaltend eine Verbindung gemäß einem der Ansprüche 1-8 oder deren physiologisch verträgliches Salz.

**11.** Verbindung gemäß einem der Ansprüche 1-8 zur Anwendung als Heilmittel.

**12.** Verbindung gemäß einem der Ansprüche 1-8 zur Anwendung als Heilmittel bei der Behandlung des Bluthochdrucks.

**Patentansprüche für folgenden Vertragsstaat : AT**

**1.** Verfahren zur Herstellung einer Verbindung der Formel 1b

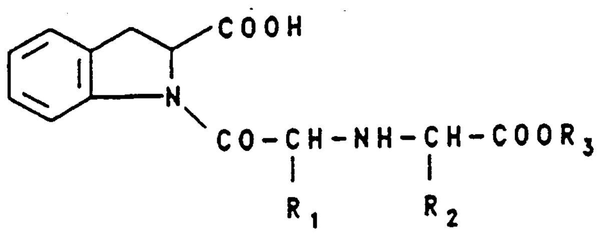

(Ib)

in welcher
$R_1$ und $R_2$ gleich oder verschieden sind und
a) Alkyl oder Alkenyl mit bis zu 6 C-Atomen bedeuten, welche jeweils gegebenenfalls substituiert sind durch einen oder mehrere gleiche oder verschiedene Substituenten aus der Reihe
1. $(C_5-C_7)$-Cycloalkyl,
2. $(C_5-C_7)$-Cycloalkenyl,
3. Hydroxy,
4. Aryloxy, das gegebenenfalls substituiert ist durch Methoxy, Ethoxy, Carboxy, Carbamoyl, Amino, $(C_1-C_6)$-Alkylamino oder durch Halogen und/oder Nitro,
5. Amino,
6. Monoalkylamino bis zu 7 C-Atomen, das im Alkylrest gegebenenfalls substituiert ist durch Hydroxy, Carboxy, Carbamoyl, Ethoxycarbonyl, Amino, $(C_1-C_6)$-Alkylamino, Di-$(C_1-C_6)$-alkylamino, Piperidino oder Morpholino,
7. Dialkylamino mit bis zu 7 C-Atomen, das im Alkylrest gegebenenfalls wie unter a) 6 beschrieben substituiert ist,
8. Cycloalkylamino mit bis zu 7 C-Atomen, das im Alkylrest gegebenenfalls wie unter a) 6 beschrieben substituiert ist,
9. Dicycloalkylamino mit bis zu 7 C-Atomen, das im Alkylrest gegebenenfalls wie unter a) 6 beschrieben substituiert ist,
10. $(C_1-C_6)$-Alkoxycarbonylamino,
11. Aryloxycarbonylamino, das im Arylteil gegebenenfalls mono- oder disubstituiert ist durch $(C_1-C_2)$-Alkoxy, Methylendioxy, Amino, Hydroxy, Acetoxy, Carboxy, Carbamoyl, Ethoxycarbonyl, Halogen oder Nitro,
12. Aralkyloxycarbonylamino, das im Arylteil gegebenenfalls wie unter a) 11 beschrieben mono- oder di-substituiert ist,
13. $(C_1-C_6)$-Alkylureido,
14. Arylureido, das im Arylteil gegebenenfalls wie unter a) 11 beschrieben durch gleiche oder verschiedene Reste mono- oder di-substituiert ist,
15. Aralkylureido, das im Arylteil gegebenenfalls wie unter a) 11 beschrieben mono- oder di-substituiert ist,
16. Formyl,
17. Alkanoylamino mit bis zu 10 C-Atomen,

18. Aroylamino mit bis zu 10 C-Atomen, das im Arylteil gegebenenfalls wie unter a) 11 beschrieben mono- oder disubstituiert ist,

19. Aralkanoylamino mit bis zu 10 C-Atomen, das im Arylteil gegebenenfalls wie unter a) 11 beschrieben mono- oder disubstituiert ist,

20. Arylamino, das im Arylteil gegebenenfalls wie unter a) 11 durch gleiche oder verschiedene Reste mono- oder di-substituiert ist,

21. Aralkylamino, das im Arylteil gegebenenfalls wie unter a) 11 beschrieben mono- oder disubstituiert ist,

22. Alkylmercapto, Alkylsulfinyl oder Alkylsulfonyl mit bis zu 7 C-Atomen, das jeweils im Alkylteil gegebenenfalls substituiert ist durch Methoxy, Ethoxy, Hydroxy, Carboxy, Carbamoyl, Ethoxycarbonyl, Amino oder $(C_1-C_6)$-Alkylamino,

23. Phenylmercapto, Phenylsulfinyl oder Phenylsulfonyl, das im Arylteil gegebenenfalls wie unter a) 22 beschrieben oder durch Halogen, Nitro oder Sulfamoyl substituiert ist,

24. Benzylmercapto, Benzylsulfinyl oder Benzylsulfonyl, das im Alkylteil gegebenenfalls wie unter a) 22 und das im Arylteil gegebenenfalls wie vorstehender Alkylteil oder wie unter a) 23 beschrieben substituiert ist,

25. Carboxy,

26. Carbamoyl,

27. Alkylcarbamoyl mit bis zu 6 C-Atomen,

28. Cycloalkylcarbamoyl mit bis zu 6 C-Atomen,

29. Dialkylcarbamoyl mit bis zu 6 C-Atomen,

30. Arylcarbamoyl,

31. Aralkylcarbamoyl,

32. Guanidino,

33. Phenyl, Naphthyl, Dihydronaphthyl oder Tetrahydronaphthyl die jeweils gegebenenfalls mono- oder di- substituiert sind durch Halogen, Hydroxy, Acetoxy, Carboxy, Carbamoyl, Sulfamoyl, Nitro, Methyl, Ethyl, Methoxy und/oder Ethoxy,

34. einen 5- bis 7-gliedrigen monocyclischen oder 9- bis 10-gliedrigen bicyclischen Heterocyclen-Rest, gegebenenfalls 1 bis 2 S- oder O-Atome und/oder bis zu 4 N-Atome pro Ring enthaltend, der gegebenenfalls substituiert durch Halogen, Hydroxy, Carboxy, Carbamoyl, Sulfamoyl, Nitro, Methoxy und/oder Ethoxy;

b) Cycloalkyl oder Cycloalkenyl mit je 5-7 C-Atomen bedeuten;

c) Aryl oder teilhydriertes Aryl mit 6 bis 10 C-Atomen bedeuten;

d) ein mono- oder bicyclischer Heterocyclen-Rest mit 5-7 bzw. 8-10 Gliedern, davon 1-2 -S- oder -O- und/oder bis zu 4 N-Atomen bedeuten, das gegebenenfalls wie unter a) 34 beschrieben substituiert ist und

$R_3$ Wasserstoff, Alkyl mit 1-6 C-Atomen, Alkenyl mit 2-6 C-Atomen, Aralkyl mit 7 - 14 C-Atomen inklusive p-Nitrobenzyl bedeutet,

wobei, falls im einzelnen nicht anders definiert, Aryl 6-10 C-Atome aufweist und gegebenenfalls durch Halogen, $(C_1-C_6)$-Alkyl oder $(C_1-C_6)$-Alkoxy substituiert ist, und

Aralkyl, Benzyl oder Phenethyl bedeutet und gegebenenfalls durch Halogen, $(C_1-C_6)$-Alkyl oder $(C_1-C_6)$-Alkoxy im Phenylkern substituiert ist,

oder deren physiologisch verträglichen Salze, dadurch gekennzeichnet, daß man

(a) eine Verbindung der Formel II' mit einer Verbindung der Formel III umsetzt,

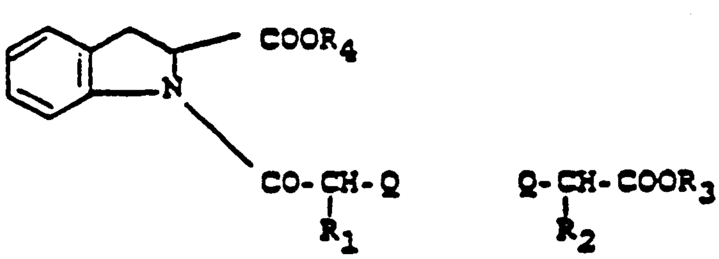

(II') (III)

in denen $R_1$, $R_2$ und $R_3$ wie oben definiert sind, ein Q eine nucleofuge Gruppe und das andere Q $-NH_2$ bedeutet und $R_4$ für H, Methyl, Ethyl, Benzyl oder tert.-Butyl steht, und gegebenenfalls einen erhaltenen Ester in die Carbonsäure ($R_2$ und/oder $R_4$ = Wasserstoff) überführt oder
(b) eine Verbindung der Formel IV

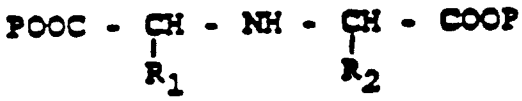

(IV)

worin $R_1$ und $R_2$ wie oben definiert sind, P H bedeutet, eines der beiden P jedoch auch die anderen Bedeutungen von $R_3$ inklusive tert.-Butyl haben kann, mit einer Verbindung der Formel V'

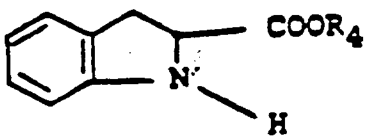

(V')

worin $R_4$ wie oben definiert ist, in Gegenwart eines Kondensationsmittels umsetzt und gegebenenfalls eine oder beide Estergruppen in die Carbonsäure überführt oder
(c) eine Verbindung der Formel VI' mit einer Verbindung der Formel VII umsetzt

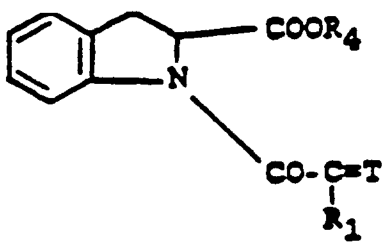

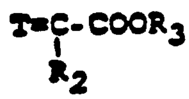

(VI') (VII)

in denen $R_1$, $R_2$, $R_3$ und $R_4$ wie oben definiert sind, ein T für ein Wasserstoffatom und eine $NH_2$-Gruppe und das andere T für ein Sauerstoffatom steht, und die erhaltene Schiff'sche Base reduziert, in den nach (a)-(c) erhaltenen Verbindungen Schwefelfunktionen gegebenenfalls zum Sulfoxid oder zum Sulfon oxidiert
und die so erhaltene Verbindung der Formel Ib gegebenenfalls in ihr Salz überführt.

**2.** Verfahren gemäß Anspruch 1, dadurch gekennzeichnet, daß eine Verbindung der Formel Ib hergestellt wird, in welcher $R_1$ Methyl und $R_2$ Phenethyl oder durch Halogen, Methyl oder Methoxy substituiertes Phenyl ist.

**3.** Verfahren gemäß Anspruch 1 oder 2, dadurch gekennzeichnet, daß eine Verbindung der Formel Ib hergestellt wird, in welcher $R_3$ Wasserstoff, Ethyl, Butyl, t-Butyl, Benzyl, p-Nitrobenzyl bedeutet.

**4.** Verfahren gemäß Anspruch 1 oder 3, dadurch gekennzeichnet, daß eine Verbindung der Formel Ib hergestellt wird, in welcher $R_1$ die Seitenkette einer natürlich vorkommenden L-Aminosäure oder deren Ethers, Esters oder Amids ist.

**5.** Verfahren gemäß Anspruch 4, dadurch gekennzeichnet, daß eine Verbindung der Formel Ib hergestellt wird, in welcher $R_1$ Methyl, Isobutyl, Methylthioethyl, Carboxymethyl, Carboxyethyl, Amino-n-butyl, Guanidino-n-propyl, Imidazol-4-ethyl, Benzyl, 4-Hydroxybenzyl oder 3-Indolmethyl bedeutet.

**6.** Verfahren gemäß Anspruch 1 oder 3, dadurch gekennzeichnet, daß eine Verbindung der Formel Ib hergestellt wird, in welcher $R_1$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl, Naphthyl, Di- oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

**7.** Verfahren gemäß Anspruch 1 oder 3, dadurch gekennzeichnet, daß eine Verbindung der Formel Ib hergestellt wird, in welcher $R_1$ Methyl, Ethyl, n-Propyl, Isopropyl, n-Butyl, Isobutyl, tert.-Butyl, geradkettiges oder verzweigtes Pentyl oder Hexyl, Cyclopentyl, Cyclohexyl, Cycloheptyl, Cyclopentenyl, Cyclohexenyl, Cycloheptenyl, Phenyl, Naphthyl, Di- oder Tetrahydronaphthyl, Benzyl, Phenethyl, p-Fluorphenethyl, o-Methylphenethyl, p-Methoxyphenethyl, 2,4-Dichlorphenethyl oder Cyclohexylethyl bedeutet.

**8.** Verfahren gemäß einem der Ansprüche 1 bis 7, dadurch gekennzeichnet, daß eine Verbindung der Formel Ib hergestellt wird, in welcher die zwei oder drei chiralen Zentren an α-C-Atomen in der L-Konfiguration vorliegen.

**9.** Verfahren zur Herstellung eines Mittels, enthaltend eine Verbindung der Formel Ib gemäß einem der Ansprüche 1 bis 8 oder deren physiologisch verträgliches Salz, dadurch gekennzeichnet, daß man diese in eine geeignete Darreichungsform bringt.

**10.** Verfahren gemäß einem der Ansprüche 1 bis 8 zur Herstellung einer Verbindung der Formel Ib zur Anwendung als Heilmittel.

**11.** Verfahren gemäß einem der Ansprüche 1 bis 8 zur Herstellung einer Verbindung der Formel Ib zur Anwendung als Heilmittel bei der Behandlung des Bluthochdrucks.

**Claims**
**Claims for the following Contracting States : BE, CH, DE, FR, GB, IT, LI, LU, NL, SE**

**1.** A compound of the formula Ib

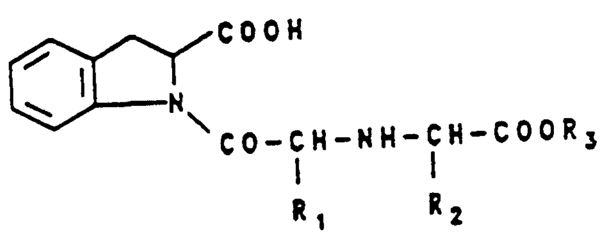

(Ib)

in which $R_1$ and $R_2$ are identical or different and

a) denote alkyl or alkenyl having up to 6 carbon atoms, each of which may be substituted by one or more identical or different substituents from the series comprising

1. $(C_5-C_7)$-cycloalkyl,
2. $(C_5-C_7)$-cycloalkenyl,
3. hydroxyl,
4. aryloxy which may be substituted by methoxy, ethoxy, carboxyl, carbamoyl, amino or $(C_1-C_6)$-alkylamino, or by halogen and/or nitro,
5. amino,
6. monoalkylamino having up to 7 carbon atoms, which may be substituted in the alkyl radical by hydroxyl, carboxyl, carbamoyl, ethoxycarbonyl, amino, $(C_1-C_6)$-alkylamino, di-$(C_1-C_6)$-alkylamino, piperidino or morpholino,
7. dialkylamino having up to 7 carbon atoms, which may be substituted in the alkyl radical as described under a) 6,
8. cycloalkylamino having up to 7 carbon atoms, which may be substituted in the alkyl radical as described under a) 6,

9. dicycloalkylamino having up to 7 carbon atoms, which may be substituted in the alkyl radical as described under a) 6,
10. ($C_1$-$C_6$)-alkoxycarbonylamino,
11. aryloxycarbonylamino which may be monosubstituted or disubstituted in the aryl moiety by ($C_1$-$C_2$)-alkoxy, methylenedioxy, amino, hydroxyl, acetoxy, carboxyl, carbamoyl, ethoxycarbonyl, halogen or nitro,
12. aralkyloxycarbonylamino which may be monosubstituted or disubstituted in the aryl moiety as described under a) 11,
13. ($C_1$-$C_6$)-alkylureido,
14. arylureido which may be monosubstituted or disubstituted in the aryl moiety by identical or different radicals as described under a) 11,
15. aralkylureido which may be monosubstituted or disubstituted in the aryl moiety as described under a) 11,
16. formyl,
17. alkanoylamino having up to 10 carbon atoms,
18. aroylamino having up to 10 carbon atoms, which may be monosubstituted or disubstituted in the aryl moiety as described under a) 11,
19. aralkanoylamino having up to 10 carbon atoms, which may be monosubstituted or disubstituted in the aryl moiety as described under a) 11,
20. arylamino which may be monosubstituted or disubstituted in the aryl moiety by identical or different radicals as described under a) 11,
21. aralkylamino which may be substituted in the aryl moiety as described under a) 11,
22. alkylmercapto, alkylsulfinyl or alkylsulfonyl having up to 7 carbon atoms, each of which may be substituted in the alkyl moiety by methoxy, ethoxy, hydroxyl, carboxyl, carbamoyl, ethoxycarbonyl, amino or ($C_1$-$C_6$)-alkylamino,
23. phenylmercapto, phenylsulfinyl or phenylsulfonyl, which may be substituted in the aryl moiety as described under a) 22 or by halogen, nitro or sulfamoyl,
24. benzylmercapto, benzylsulfinyl or benzylsulfonyl, which may be substituted in the alkyl moiety as described under a) 22 and which may be substituted in the aryl moiety as for the above alkyl moiety or as described under a) 23,
25. carboxyl,
26. carbamoyl,
27. alkylaminocarbonyl having up to 6 carbon atoms,
28. cycloalkylcarbamoyl having up to 6 carbon atoms,
29. dialkylcarbamoyl having up to 6 carbon atoms,
30. arylcarbamoyl
31. aralkylcarbamoyl,
32. guanidino,
33. phenyl, naphthyl, dihydronaphthyl or tetrahydronaphthyl, each of which may be monosubstituted or disubstituted by halogen, hydroxyl, acetoxy, carboxyl, carbamoyl, sulfamoyl, nitro, methyl, ethyl, methoxy and/or ethoxy,
34. a 5-membered to 7-membered monocyclic or 9-membered or 10-membered bicyclic heterocyclic radical which may contain 1 or 2 S or O atoms and/or up to 4 N atoms per ring and which may be substituted by halogen, hydroxyl, carboxyl, carbamoyl, sulfamoyl, nitro, methoxy and/or ethoxy;

b) denote cycloalkyl or cycloalkenyl, each having 5-7 carbon atoms;
c) denote aryl or partially hydrogenated aryl having 6 to 10 carbon atoms;
d) denote a monocyclic or bicyclic heterocyclic radical having 5-7 or 8-10 members, including 1 or 2 -S- or -O- and/or up to 4 N atoms, which radical may be substituted as described under a) 34,

and
$R_3$ denotes hydrogen, alkyl having 1 to 6 carbon atoms, alkenyl having 2 to 6 carbon atoms, aralkyl having 7 to 14 carbon atoms, including p-nitrobenzyl,
and, unless specifically defined otherwise, aryl has 6 to 10 carbon atoms and may be substituted by halogen, ($C_1$-$C_6$)-alkyl or ($C_1$-$C_6$)-alkoxy, and aralkyl denotes benzyl or phenethyl and may be substituted by halogen, ($C_1$-$C_6$)-alkyl or ($C_1$-$C_6$)-alkoxy in the phenyl nucleus,
or its physiologically tolerated salts.

2. A compound as claimed in claim 1, wherein $R_1$ is methyl and $R_2$ is phenethyl or phenethyl which is substituted by halogen, methyl or methoxy.

3. A compound as claimed in claim 1 or 2, wherein $R_3$ is hydrogen, ethyl, butyl, t-butyl, benzyl or p-nitrobenzyl.

4. A compound as claimed in claim 1 or 3, wherein $R_1$ denotes the side chain of a naturally occurring L-amino acid or its ether, ester or amide.

5. A compound as claimed in claim 4, wherein $R_1$ is methyl, isobutyl, methylthioethyl, carboxymethyl, carboxyethyl, amino-n-butyl, guanidino-n-propyl, imidazole-4-ethyl, benzyl, 4-hydroxybenzyl or 3-indolemethyl.

6. A compound as claimed in claim 1 or 3, wherein $R_1$ is methyl, ethyl, n-propyl, isopropyl, n-butyl, isobutyl, tert-butyl, straight-chain or branched pentyl or hexyl, cyclopentyl, cyclohexyl, cycloheptyl, cyclopentenyl, cyclohexenyl, cycloheptenyl, phenyl, naphthyl, di- or tetrahydronaphthyl, benzyl, phenethyl, p-fluorophenethyl, o-methylphenethyl, p-methoxyphenethyl, 2,4-dichlorophenethyl or cyclohexylethyl.

7. A compound as claimed in claim 1 or 3, wherein $R_2$ is methyl, ethyl, n-propyl, isopropyl, n-butyl, isobutyl, tert-butyl, straight-chain or branched pentyl or hexyl, cyclopentyl, cyclohexyl, cycloheptyl, cyclopentenyl, cyclohexenyl, cycloheptenyl, phenyl, naphthyl, di- or tetrahydronaphthyl, benzyl, phenethyl, p-fluorophenethyl, o-methylphenethyl, p-methoxyphenethyl, 2,4-dichlorophenethyl or cyclohexylethyl.

8. A compound as claimed in any of claims 1 to 7, wherein the two or three chiral centers are present at $\alpha$ carbon atoms in the L configuration.

9. A process for the preparation of a compound as claimed in any one of claims 1 to 8, which comprises
a) reacting a compound of the formula II' with a compound of the formula III

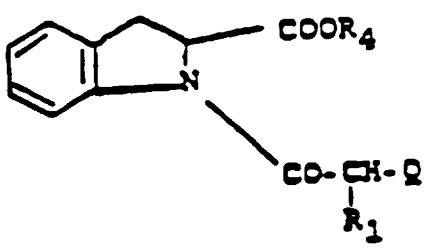

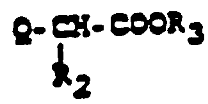

(II') (III)

in which $R_1$, $R_2$ and $R_3$ are as defined in claim 1, one Q denotes a nucleofugic group and the other Q denotes $-NH_2$, and $R_4$ represents H, methyl, ethyl, benzyl or tert-butyl, and, if appropriate, converting an ester obtained into the carboxylic acid ($R_2$ and/or $R_4$ = hydrogen), or
b) reacting a compound (IV)

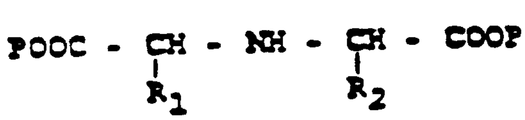

(IV)

wherein $R_1$ and $R_2$ are as defined in claim 1, P denotes H, but one of the two Ps can also have the other meanings of $R_3$ in claim 1 including tert-butyl, with a compound of the formula V'

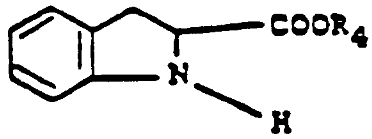

(V')

in which $R_4$ is as defined above, in the presence of a condensing agent, and, if appropriate, converting one or both ester groups into the carboxylic acid, or

c) reacting a compound of the formula VI' with a compound of the formula VII

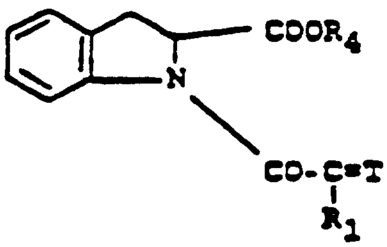

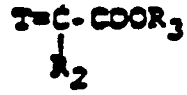

(VI') (VII)

in which $R_1$, $R_2$ and $R_3$ are as defined in claim 1 and $R_4$ is as defined above, one T represents a hydrogen atom and an $NH_2$ group and the other T represents an oxygen atom, and reducing the Schiff's base obtained, if required oxidizing sulfur functions in the compound obtained according to a) to c) to the sulfoxide or to the sulfone, and, if appropriate, converting the resulting compound of the formula Ib into its salt.

**10.** A composition containing a compound as claimed in any one of claims 1 to 8 or its physiologically tolerated salt.

**11.** A compound as claimed in any one of claims 1 to 8 for use as a medicament.

**12.** A compound as claimed in any one of claims 1 to 8 for use as a medicament in the treatment of high blood pressure.

**Claims for the following Contracting State : AT**

**1.** A process for the preparation of a compound of the formula Ib

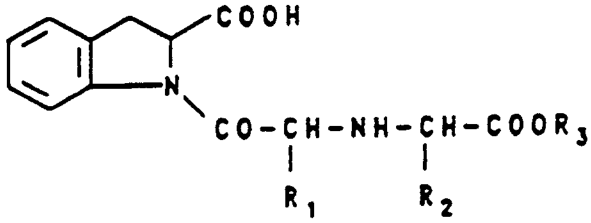

(Ib)

in which $R_1$ and $R_2$ are identical or different and

a) denote alkyl or alkenyl having up to 6 carbon atoms, each of which may be substituted by one or more identical or different substituents from the series comprising

1. ($C_5$-$C_7$)-cycloalkyl,
2. ($C_5$-$C_7$)-cycloalkenyl,
3. hydroxyl,

4. aryloxy which may be substituted by methoxy, ethoxy, carboxyl, carbamoyl, amino or ($C_1$-$C_6$)-alkylamino, or by halogen and/or nitro,
5. amino,
6. monoalkylamino having up to 7 carbon atoms, which may be substituted in the alkyl radical by hydroxyl, carboxyl, carbamoyl, ethoxycarbonyl, amino, ($C_1$-$C_6$)-alkylamino, di-($C_1$-$C_6$)-alkylamino, piperidino or morpholino,
7. dialkylamino having up to 7 carbon atoms, which may be substituted in the alkyl radical as described under a) 6,
8. cycloalkylamino having up to 7 carbon atoms, which may be substituted in the alkyl radical as described under a) 6,
9. dicycloalkylamino having up to 7 carbon atoms, which may be substituted in the alkyl radical as described under a) 6,
10. ($C_1$-$C_6$)-alkoxycarbonylamino,
11. aryloxycarbonylamino which may be monosubstituted or disubstituted in the aryl moiety by ($C_1$-$C_2$)-alkoxy, methylenedioxy, amino, hydroxyl, acetoxy, carboxyl, carbamoyl, ethoxycarbonyl, halogen or nitro,
12. aralkyloxycarbonylamino which may be monosubstituted or disubstituted in the aryl moiety as described under a) 11,
13. ($C_1$-$C_6$)-alkylureido,
14. arylureido which may be monosubstituted or disubstituted in the aryl moiety by identical or different radicals as described under a) 11,
15. aralkylureido which may be monosubstituted or disubstituted in the aryl moiety as described under a) 11,
16. formyl,
17. alkanoylamino having up to 10 carbon atoms,
18. aroylamino having up to 10 carbon atoms, which may be monosubstituted or disubstituted in the aryl moiety as described under a) 11,
19. aralkanoylamino having up to 10 carbon atoms, which may be monosubstituted or disubstituted in the aryl moiety as described under a) 11,
20. arylamino which may be monosubstituted or disubstituted in the aryl moiety by identical or different radicals as described under a) 11,
21. aralkylamino which may be substituted in the aryl moiety as described under a) 11,
22. alkylmercapto, alkylsulfinyl or alkylsulfonyl having up to 7 carbon atoms, each of which may be substituted in the alkyl moiety by methoxy, ethoxy, hydroxyl, carboxyl, carbamoyl, ethoxycarbonyl, amino or ($C_1$-$C_6$)-alkylamino,
23. phenylmercapto, phenylsulfinyl or phenylsulfonyl, which may be substituted in the aryl moiety as described under a) 22 or by halogen, nitro or sulfamoyl,
24. benzylmercapto, benzylsulfinyl or benzylsulfonyl, which may be substituted in the alkyl moiety as described under a) 22 and which may be substituted in the aryl moiety as for the above alkyl moiety or as described under a) 23,
25. carboxyl,
26. carbamoyl,
27. alkylaminocarbonyl having up to 6 carbon atoms,
28. cycloalkylcarbamoyl having up to 6 carbon atoms,
29. dialkylcarbamoyl having up to 6 carbon atoms,
30. arylcarbamoyl
31. aralkylcarbamoyl,
32. guanidino,
33. phenyl, naphthyl, dihydronaphthyl or tetrahydronaphthyl, each of which may be monosubstituted or disubstituted by halogen, hydroxyl, acetoxy, carboxyl, carbamoyl, sulfamoyl, nitro, methyl, ethyl, methoxy and/or ethoxy,
34. a 5-membered to 7-membered monocyclic or 9-membered or 10-membered bicyclic heterocyclic radical which may contain 1 or 2 S or O atoms and/or up to 4 N atoms per ring and which may be substituted by halogen, hydroxyl, carboxyl, carbamoyl, sulfamoyl, nitro, methoxy and/or ethoxy;

b) denote cycloalkyl or cycloalkenyl, each having 5-7 carbon atoms;
c) denote aryl or partially hydrogenated aryl having 6 to 10 carbon atoms;

d) denote a monocyclic or bicyclic heterocyclic radical having 5-7 or 8-10 members, including 1 or 2 -S- or -O- and/or up to 4 N atoms, which radical may be substituted as described under a) 34,

and

$R_3$ denotes hydrogen, alkyl having 1 to 6 carbon atoms, alkenyl having 2 to 6 carbon atoms, aralkyl having 7 to 14 carbon atoms, including p-nitrobenzyl,

and, unless specifically defined otherwise, aryl has 6 to 10 carbon atoms and may be substituted by halogen, $(C_1-C_6)$-alkyl or $(C_1-C_6)$-alkoxy, and aralkyl denotes benzyl or phenethyl and may be substituted by halogen, $(C_1-C_6)$-alkyl or $(C_1-C_6)$-alkoxy in the phenyl nucleus,

or its physiologically tolerated salts, which comprises

a) reacting a compound of the formula II' with a compound of the formula III

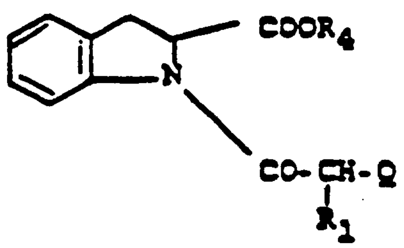

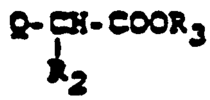

(II') (III)

in which $R_1$, $R_2$ and $R_3$ are as defined in claim 1, Q denotes a nucleofugic group and the other Q denotes $-NH_2$, and $R_4$ represents H, methyl, ethyl, benzyl or tert-butyl, and, if appropriate, converting an ester obtained into the carboxylic acid ($R_2$ and/or $R_4$ = hydrogen), or

b) reacting a compound (IV)

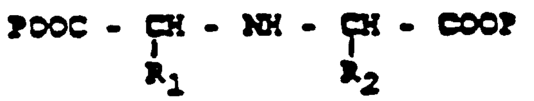

(IV)

wherein $R_1$ and $R_2$ are as defined in claim 1, P denotes H, but one of the two Ps can also have the other meanings of $R_3$ in claim 1 including tert-butyl, with a compound of the formula V'

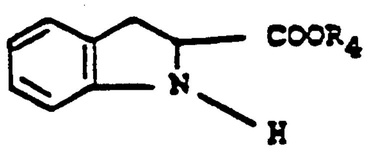

(V')

in which $R_4$ is as defined above, in the presence of a condensing agent, and, if appropriate, converting one or both ester groups into the carboxylic acid, or

c) reacting a compound of the formula VI' with a compound of the formula VII

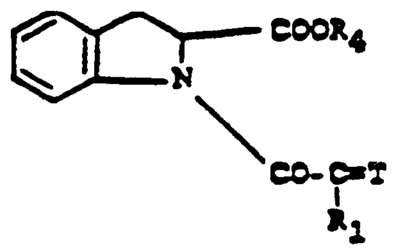

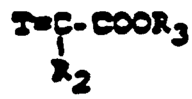

(VI') (VII)

in which $R_1$, $R_2$, $R_3$ and $R_4$ are as defined above, one T represents a hydrogen atom and an $NH_2$ group and the other T represents an oxygen atom, and reducing the Schiff's base obtained, if required oxidizing sulfur functions in the compound obtained according to a) to c) to the sulfoxide or to the sulfone, and, if appropriate, converting the resulting compound of the formula Ib into its salt.

**2.** The process as claimed in claim 1, wherein a compound of the formula Ib is prepared in which $R_1$ is methyl and $R_2$ is phenethyl or phenethyl which is substituted by halogen, methyl or methoxy.

**3.** The process as claimed in claim 1 or 2, wherein a compound of the formula Ib is prepared in which $R_3$ is hydrogen, ethyl, butyl, t-butyl, benzyl or p-nitrobenzyl.

**4.** The process as claimed in claim 1 or 3, wherein a compound of the formula Ib is prepared in which $R_1$ denotes the side chain of a naturally occurring L-amino acid or its ether, ester or amide.

**5.** The process as claimed in claim 4, wherein a compound of the formula Ib is prepared in which $R_1$ is methyl, isobutyl, methylthioethyl, carboxymethyl, carboxyethyl, amino-n-butyl, guanidino-n-propyl, imidazole-4-ethyl, benzyl, 4-hydroxybenzyl or 3-indolemethyl.

**6.** The process as claimed in claim 1 or 3, wherein a compound of the formula Ib is prepared in which $R_1$ is methyl, ethyl, n-propyl, isopropyl, n-butyl, isobutyl, tert-butyl, straight-chain or branched pentyl or hexyl, cyclopentyl, cyclohexyl, cycloheptyl, cyclopentenyl, cyclohexenyl, cycloheptenyl, phenyl, naphthyl, di- or tetrahydronaphthyl, benzyl, phenethyl, p-fluorophenethyl, o-methylphenethyl, p-methoxyphenethyl, 2,4-dichlorophenethyl or cyclohexylethyl.

**7.** The process as claimed in claim 1 or 3, wherein a compound of the formula Ib is prepared in which $R_2$ is methyl, ethyl, n-propyl, isopropyl, n-butyl, isobutyl, tert-butyl, straight-chain or branched pentyl or hexyl, cyclopentyl, cyclohexyl, cycloheptyl, cyclopentenyl, cyclohexenyl, cycloheptenyl, phenyl, naphthyl, di- or tetrahydronaphthyl, benzyl, phenethyl, p-fluorophenethyl, o-methylphenethyl, p-methoxyphenethyl, 2,4-dichlorophenethyl or cyclohexylethyl.

**8.** The process as claimed in any of claims 1 to 7, wherein a compound of the formula Ib is prepared in which the two or three chiral centers are present at $\alpha$ carbon atoms in the L configuration.

**9.** A process for the preparation of a composition containing a compound of the formula Ib as in any of claims 1 to 8 or its physiologically tolerated salt, which comprises bringing this compound or salt into a suitable administration form.

**10.** The process as claimed in any of claims 1 to 8 for the preparation of a compound of the formula Ib for use as a medicament.

**11.** The process as claimed in any of claims 1 to 8 for the preparation of a medicament in the treatment of high blood pressure.

**Revendications**

**Revendications pour les Etats contractants suivants : BE, CH, DE, FR, GB, IT, LI, LU, NL, SE**

**1.** Composé de formule Ib

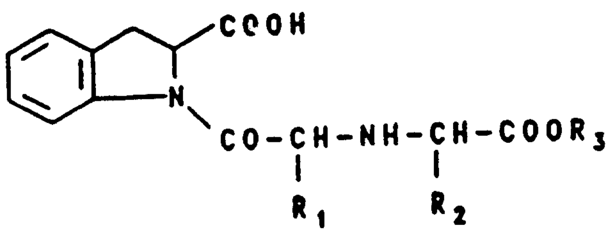

(Ib)

dans laquelle

$R_1$ et $R_2$ sont identiques ou différents et représentent

a) un radical alkyle ou alcényle ayant jusqu'à 6 atomes de carbone, qui sont éventuellement substitués chacun par un ou plusieurs substituants, identiques ou différents, choisis parmi des radicaux

1. cycloalkyle en $C_5$-$C_7$,
2. cycloalcényle en $C_5$-$C_7$,
3. hydroxy,
4. aryloxy, qui est éventuellement substitué par des atomes d'halogène ou par des groupes méthoxy, éthoxy, carboxy, carbamoyle, amino, alkyl($C_1$-$C_6$)-amino et/ou nitro,
5. amino,
6. monoalkylamino ayant jusqu'à 7 atomes de carbone, qui est éventuellement substitué sur le fragment alkyle par des groupes hydroxy, carboxy, carbamoyle, éthoxycarbonyle, amino, alkyl-($C_1$-$C_6$)-amino, dialkyl($C_1$-$C_6$)-amino, pipéridino ou morpholino,
7. dialkyleamino ayant jusqu'à 7 atomes de carbone, qui est éventuellement substitué sur le fragment alkyle comme décrit en a)6,
8. cycloalkylamino ayant jusqu'à 7 atomes de carbone, qui est éventuellement substitué sur le fragment alkyle comme décrit en a)6,
9. dicycloalkylamino ayant jusqu'à 7 atomes de carbone, qui est éventuellement substitué sur le fragment alkyle comme décrit en a)6,
10. alcoxy($C_1$-$C_6$)-carbonylamino,
11. aryloxycarbonylamino, qui est éventuellement mono- ou disubstitué sur le fragment aryle par un ou des atomes d'halogène ou groupes alcoxy en $C_1$-$C_2$, méthylènedioxy, amino, hydroxy, acétoxy, carboxy, carbamoyle, éthoxycarbonyle ou nitro,
12. aralkyloxycarbonylamino, qui est éventuellement mono- ou disubstitué sur le fragment aryle comme décrit en a)11,
13. alkyl($C_1$-$C_6$)-uréido,
14. aryluréido, qui est éventuellement mono- ou disubstitué sur le fragment aryle par des groupes identiques ou différents, comme décrit en a)11,
15. aralkyluréido, qui est éventuellement mono- ou disubstitué sur le fragment aryle comme décrit en a)11,
16. formyle,
17. alcanoylamino ayant jusqu'à 10 atomes de carbone,
18. aroylamino ayant jusqu'à 10 atomes de carbone, qui est éventuellement mono- ou disubstitué sur le fragment aryle comme décrit en a)11,
19. aralkanoylamino ayant jusqu'à 10 atomes de carbone, qui est éventuellement mono- ou disubstitué sur le fragment aryle comme décrit en a)11,
20. arylamino, qui est éventuellement mono- ou disubstitué sur le fragment aryle par des groupes identiques ou différents comme en a)11,
21. aralkylamino, qui est éventuellement mono- ou disubstitué sur le fragment aryle comme décrit en a)11,
22. alkylmercapto, alkylsulfinyle ou alkylsulfonyle ayant jusqu'à 7 atomes de carbone, qui sont éventuellement substitués chacun sur le fragment alkyle par des groupes méthoxy, éthoxy,

hydroxy, carboxy, carbamoyle, éthoxycarbonyle, amino ou alkyl($C_1$-$C_6$)-amino,
23. phénylmercapto, phénylsulfinyle ou phénylsulfonyle, qui est éventuellement substitué sur le fragment aryle, comme décrit en a)22, ou par des atomes d'halogène ou par le groupe nitro ou sulfamoyle,
24. benzylmercapto, benzylsulfinyle ou benzylsulfonyle, qui est éventuellement substitué sur le fragment alkyle comme en a)22 et qui est éventuellement substitué sur le fragment aryle comme le fragment alkyle précédent ou comme décrit en a)23,
25. carboxy,
26. carbamoyle,
27. alkylcarbamoyle ayant jusqu'à 6 atomes de carbone,
28. cycloalkylcarbamoyle ayant jusqu'à 6 atomes de carbone,
29. dialkylcarbamoyle ayant jusqu'à 6 atomes de carbone,
30. arylcarbamoyle,
31. aralkylcarbamoyle,
32. guanidino,
33. phényle, naphtyle, dihydronaphtyle ou tétrahydronaphtyle, qui sont éventuellement mono- ou disubstitués chacun par un ou des atomes d'halogène ou groupes hydroxy, acétoxy, carboxy, carbamoyle, sulfamoyle , nitro, méthyle, éthyle, méthoxy et/ou éthoxy,
34. un cycle hétérocyclique monocyclique à 5-7 chaînons ou bicyclique à 9-10 chaînons, contenant éventuellement 1 ou 2 atomes de S ou O et/ou jusqu'à 4 atomes de N par cycle, et éventuellement substitué par des atomes d'halogène ou des groupes hydroxy, carboxy, carbamoyle, sulfamoyle, nitro, méthoxy et/ou éthoxy;

b) un radical cycloalkyle ou cycloalcényle ayant chacun 5 à 7 atomes de carbone;
c) un radical aryle ou aryle partiellement hydrogéné ayant de 6 à 10 atomes de carbone;
d) un radical hétérocyclique mono- ou bicyclique ayant 5-7 ou, respectivement, 8-10 chaînons, dont 1-2 représentent des atomes de S ou O et/ou jusqu'à 4 atomes de N, qui est éventuellement substitué comme décrit en a)34; et

$R_3$ représente un atome d'hydrogène ou un radical alkyle ayant de 1 à 6 atomes de carbone, alcényle ayant de 2 à 6 atomes de carbone, aralkyle ayant de 7 à 14 atomes de carbone, y compris le radical p-nitrobenzyle,
a moins d'indication contraire, le radical aryle comportant 6 à 10 atomes de carbone et étant éventuellement substitué par des atomes d'halogène ou des groupes alkyle en $C_1$-$C_6$ ou alcoxy en $C_1$-$C_6$, et
le radical aralkyle représentant le radical benzyle ou phénéthyle et étant éventuellement substitué sur le noyau phényle par des atomes d'halogène ou des groupes alkyle en $C_1$-$C_6$ ou alcoxy en $C_1$-$C_6$,
ou sels physiologiquement acceptables de celui-ci.

**2.** Composé selon la revendication 1, dans lequel $R_1$ est le radical méthyle et $R_2$ est le radical phénéthyle ou un radical phénéthyle substitué par un atome d'halogène ou par le groupe méthyle ou méthoxy.

**3.** Composé selon la revendication 1 ou 2, dans lequel $R_3$ représente un atome d'hydrogène ou le groupe éthyle, butyle, tert-butyle, benzyle, p-nitrobenzyle.

**4.** Composé selon la revendication 1 ou 3, dans lequel $R_1$ est la chaîne latérale d'un L-aminoacide existant dans la nature ou un éther, ester ou amide de celui-ci.

**5.** Composé selon la revendication 4, dans lequel $R_1$ représente le radical méthyle, isobutyle, méthylthioéthyle, carboxyméthyle, carboxyéthyle, amino-n-butyle, guanidino-n-propyle, imidazol-4-éthyle, benzyle, 4-hydroxybenzyle ou 3-indol-méthyle.

**6.** Composé selon la revendication 1 ou 3, dans lequel $R_1$ représente le radical méthyle, éthyle, n-propyle, isopropyle, n-butyle, isobutyle, tert-butyle, pentyle ou hexyle à chaîne droite ou ramifiée, cyclopentyle, cyclohexyle, cycloheptyle, cyclopentényle, cyclohexényle, cycloheptényle, phényle, naphtyle, di- ou tétrahydronaphtyle, benzyle, phénéthyle, p-fluorophénéthyle, o-méthylphénéthyle, p-méthoxyphénéthyle, 2,4-dichlorophénéthyle ou cyclohexyléthyle.

**7.** Composé selon la revendication 1 ou 3, dans lequel $R_2$ représente le radical méthyle, éthyle, n-propyle, isopropyle, n-butyle, isobutyle, tert-butyle, pentyle ou hexyle à chaîne droite ou ramifiée, cyclopentyle,

cyclohexyle, cycloheptyle, cyclopentényle, cyclohexényle, cycloheptényle, phényle, naphtyle, di- ou tétrahydronaphtyle, benzyle, phénéthyle, p-fluorophénéthyle, o-méthylphénéthyle, p-méthoxyphénéthyle, 2,4-dichlorophénéthyle ou cyclohexyléthyle.

**8.** Composé selon l'une des revendications 1 à 7, dans lequel les deux ou trois centres chiraux au niveau des atomes de carbone α se présentent en la configuration L.

**9.** Procédé pour la préparation d'un composé selon l'une des revendications 1 à 8, caractérisé en ce que
(a) on fait réagir un composé de formule II'avec un composé de formule III

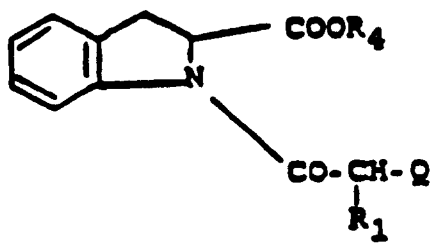

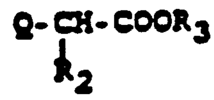

(II') (III)

formules dans lesquelles $R_1$, $R_2$ et $R_3$ sont tels que définis dans la revendication 1, un radical Q représente un groupe nucléofuge et l'autre représente Q-$NH_2$, et $R_4$ représente H, le groupe méthyle, éthyle, benzyle ou tert-butyle, et éventuellement on convertit un ester obtenu en l'acide carboxylique ($R_2$ et/ou $R_4$ = H) ou
(b) on fait réagir un composé (IV)

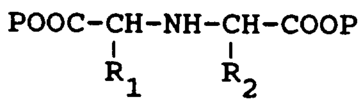

(IV)

dans lequel $R_1$ et $R_2$ sont tels que définis dans la revendication 1, P représente H, mais l'un des deux radicaux P peut également avoir les autres significations de $R_3$ dans la revendication 1, y compris celle du radical tert-butyle,
avec un composé de formule V'

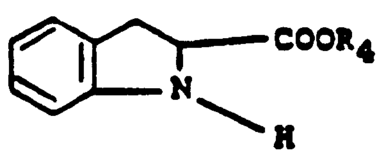

(V')

dans laquelle $R_4$ est tel que défini plus haut, en présence d'un agent de condensation, et éventuellement on convertit un groupe ester ou les deux en l'acide carboxylique, ou

(c) on fait réagir un composé de formule VI'avec un composé de formule VII

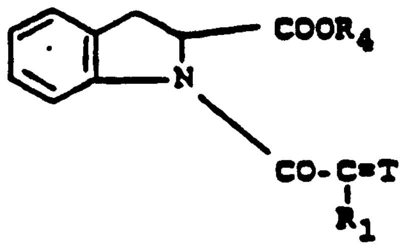

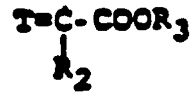

(VI') (VII)

formules dans lesquelles $R_1$, $R_2$ et $R_3$ sont tels que définis dans la revendication 1 et $R_4$ est tel que défini plus haut, un radical T représente un atome d'hydrogène et un groupe $NH_2$ et l'autre radical T représente un atome d'oxygène, et on réduit la base de Schiff obtenue, éventuellement on oxyde, dans les composés obtenus selon (a) à (c), des fonctions S en sulfoxyde ou en sulfone, et éventuellement on convertit en un de ses sels le composé de formule Ib ainsi obtenu.

**10.** Produit contenant un composé selon l'une des revendications 1 à 8 ou un sel physiologiquement acceptable de celui-ci.

**11.** Composé selon l'une des revendications 1 à 8, pour utilisation en tant que médicament.

**12.** Composé selon l'une des revendications 1 à 8, pour utilisation en tant que médicament dans le traitement de l'hypertension artérielle.

**Revendications pour l'Etat contractant suivant : AT**

**1.** Procédé pour la préparation d'un composé de formule Ib

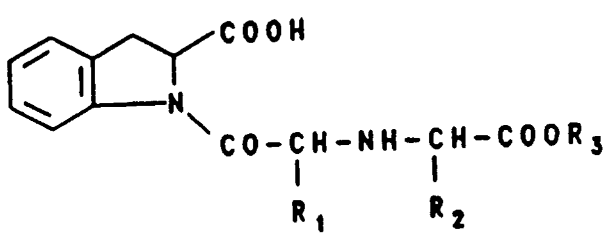

(Ib)

dans laquelle
$R_1$ et $R_2$ sont identiques ou différents et représentent
a) un radical alkyle ou alcényle ayant jusqu'à 6 atomes de carbone, qui sont éventuellement substitués chacun par un ou plusieurs substituants, identiques ou différents, choisis parmi des radicaux
1. cycloalkyle en $C_5$-$C_7$,
2. cycloalcényle en $C_5$-$C_7$,
3. hydroxy,
4. aryloxy, qui est éventuellement substitué par des atomes d'halogène ou par des groupes méthoxy, éthoxy, carboxy, carbamoyle, amino, alkyl($C_1$-$C_6$)-amino et/ou nitro,
5. amino,
6. monoalkylamino ayant jusqu'à 7 atomes de carbone, qui est éventuellement substitué sur le fragment alkyle par des groupes hydroxy, carboxy, carbamoyle, éthoxycarbonyle, amino, alkyl-($C_1$-$C_6$)-amino, dialkyl($C_1$-$C_6$)-amino, pipéridino ou morpholino,

7. dialkyleamino ayant jusqu'à 7 atomes de carbone, qui est éventuellement substitué sur le fragment alkyle comme décrit en a)6,
8. cycloalkylamino ayant jusqu'à 7 atomes de carbone, qui est éventuellement substitué sur le fragment alkyle comme décrit en a)6,
9. dicycloalkylamino ayant jusqu'à 7 atomes de carbone, qui est éventuellement substitué sur le fragment alkyle comme décrit en a)6,
10. alcoxy($C_1$-$C_6$)-carbonylamino,
11. aryloxycarbonylamino, qui est éventuellement mono- ou disubstitué sur le fragment aryle par un ou des atomes d'halogène ou groupes alcoxy en $C_1$-$C_2$, méthylènedioxy, amino, hydroxy, acétoxy, carboxy, carbamoyle, éthoxycarbonyle ou nitro,
12. aralkyloxycarbonylamino, qui est éventuellement mono- ou disubstitué sur le fragment aryle comme décrit en a)11,
13. alkyl($C_1$-$C_6$)-uréido,
14. aryluréido, qui est éventuellement mono- ou disubstitué sur le fragment aryle par des groupes identiques ou différents, comme décrit en a)11,
15. aralkyluréido, qui est éventuellement mono- ou disubstitué sur le fragment aryle comme décrit en a)11,
16. formyle,
17. alcanoylamino ayant jusqu'à 10 atomes de carbone,
18. aroylamino ayant jusqu'à 10 atomes de carbone, qui est éventuellement mono- ou disubstitué sur le fragment aryle comme décrit en a)11,
19. aralkanoylamino ayant jusqu'à 10 atomes de carbone, qui est éventuellement mono- ou disubstitué sur le fragment aryle comme décrit en a)11,
20. arylamino, qui est éventuellement mono- ou disubstitué sur le fragment aryle par des groupes identiques ou différents comme en a)11,
21. aralkylamino, qui est éventuellement mono- ou disubstitué sur le fragment aryle comme décrit en a)11,
22. alkylmercapto, alkylsulfinyle ou alkylsulfonyle ayant jusqu'à 7 atomes de carbone, qui sont éventuellement substitués chacun sur le fragment alkyle par des groupes méthoxy, éthoxy, hydroxy, carboxy, carbamoyle, éthoxycarbonyle, amino ou alkyl($C_1$-$C_6$)-amino,
23. phénylmercapto, phénylsulfinyle ou phénylsulfonyle, qui est éventuellement substitué sur le fragment aryle, comme décrit en a)22, ou par des atomes d'halogène ou par le groupe nitro ou sulfamoyle,
24. benzylmercapto, benzylsulfinyle ou benzylsulfonyle, qui est éventuellement substitué sur le fragment alkyle comme en a)22 et qui est éventuellement substitué sur le fragment aryle comme le fragment alkyle précédent ou comme décrit en a)23,
25. carboxy,
26. carbamoyle,
27. alkylcarbamoyle ayant jusqu'à 6 atomes de carbone,
28. cycloalkylcarbamoyle ayant jusqu'à 6 atomes de carbone,
29. dialkylcarbamoyle ayant jusqu'à 6 atomes de carbone,
30. arylcarbamoyle,
31. aralkylcarbamoyle,
32. guanidino,
33. phényle, naphtyle, dihydronaphtyle ou tétrahydronaphtyle, qui sont éventuellement mono- ou disubstitués chacun par un ou des atomes d'halogène ou groupes hydroxy, acétoxy, carboxy, carbamoyle, sulfamoyle , nitro, méthyle, éthyle, méthoxy et/ou éthoxy,
34. un cycle hétérocyclique monocyclique à 5-7 chaînons ou bicyclique à 9-10 chaînons, contenant éventuellement 1 ou 2 atomes de S ou O et/ou jusqu'à 4 atomes de N par cycle, et éventuellement substitué par des atomes d'halogène ou des groupes hydroxy, carboxy, carbamoyle, sulfamoyle, nitro, méthoxy et/ou éthoxy;

b) un radical cycloalkyle ou cycloalcényle ayant chacun 5 à 7 atomes de carbone;
c) un radical aryle ou aryle partiellement hydrogéné ayant de 6 à 10 atomes de carbone;
d) un radical hétérocyclique mono- ou bicyclique ayant 5-7 ou, respectivement, 8-10 chaînons, dont 1-2 représentent des atomes de S ou O et/ou jusqu'à 4 atomes de N, qui est éventuellement substitué comme décrit en a)34; et

$R_3$ représente un atome d'hydrogène ou un radical alkyle ayant de 1 à 6 atomes de carbone, alcényle ayant de 2 à 6 atomes de carbone, aralkyle ayant de 7 à 14 atomes de carbone, y compris le

radical p-nitrobenzyle,
a moins d'indication contraire, le radical aryle comportant 6 à 10 atomes de carbone et étant éventuellement substitué par des atomes d'halogène ou des groupes alkyle en $C_1$-$C_6$ ou alcoxy en $C_1$-$C_6$, et
le radical aralkyle représentant le radical benzyle ou phénéthyle et étant éventuellement substitué sur le noyau phényle par des atomes d'halogène ou des groupes alkyle en $C_1$-$C_6$ ou alcoxy en $C_1$-$C_6$,
ou de ses sels physiologiquement acceptables, caractérisé en ce que

(a) on fait réagir un composé de formule II' avec un composé de formule III

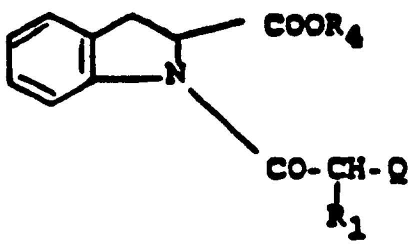
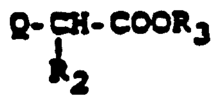

(II') (III)

formules dans lesquelles $R_1$, $R_2$ et $R_3$ sont tels que définis plus haut, un radical Q représente un groupe nucléofuge et l'autre représente Q-$NH_2$, et $R_4$ représente H, le groupe méthyle, éthyle, benzyle ou tert-butyle, et éventuellement on convertit un ester obtenu en l'acide carboxylique ($R_2$ et/ou $R_4$ = H) ou

(b) on fait réagir un composé (IV)

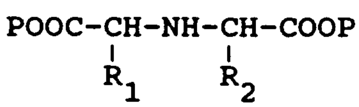

(IV)

dans lequel $R_1$ et $R_2$ sont tels que définis plus haut, P représente H, mais l'un des deux radicaux P peut également avoir les autres significations de $R_3$ plus haut, y compris celle du radical tert-butyle, avec un composé de formule V'

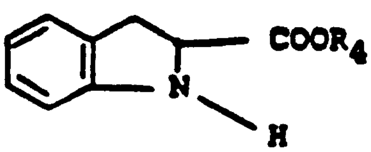

(V')

dans laquelle $R_4$ est tel que défini plus haut, en présence d'un agent de condensation, et éventuellement on convertit un groupe ester ou les deux en l'acide carboxylique, ou

(c) on fait réagir un composé de formule VI' avec un composé de formule VII

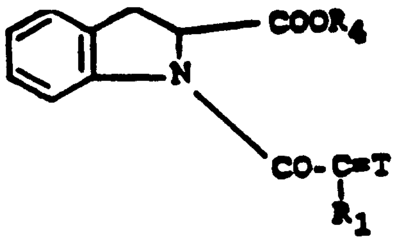

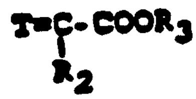

(VI') (VII)

formules dans lesquelles $R_1$, $R_2$, $R_3$ et $R_4$ sont tels que définis plus haut, un radical T représente un atome d'hydrogène et un groupe $NH_2$ et l'autre radical T représente un atome d'oxygène, et on réduit la base de Schiff obtenue, éventuellement on oxyde, dans les composés obtenus selon (a) à (c),des fonctions S en sulfoxyde ou en sulfone, et éventuellement on convertit en un de ses sels le composé de formule Ib ainsi obtenu.

2. Procédé selon la revendication 1, caractérisé en ce que l'on prépare un composé de formule Ib dans lequel $R_1$ est le radical méthyle et $R_2$ est le radical phénéthyle ou un radical phénéthyle substitué par un atome d'halogène ou par le groupe méthyle ou méthoxy.

3. Procédé selon la revendication 1 ou 2, caractérisé en ce que l'on prépare un composé de formule Ib dans lequel $R_3$ représente un atome d'hydrogène ou le groupe éthyle, butyle, tert-butyle, benzyle, p-nitrobenzyle.

4. Procédé selon la revendication 1 ou 3, caractérisé en ce que l'on prépare un composé de formule Ib dans lequel $R_1$ est la chaîne latérale d'un L-aminoacide existant dans la nature ou un éther, ester ou amide de celui-ci.

5. Procédé selon la revendication 4, caractérisé en ce que l'on prépare un composé de formule Ib dans lequel $R_1$ représente le radical méthyle, isobutyle, méthylthioéthyle, carboxyméthyle, carboxyéthyle, amino-n-butyle, guanidino-n-propyle, imidazol-4-éthyle, benzyle, 4-hydroxybenzyle ou 3-indol-méthyle.

6. Procédé selon la revendication 1 ou 3, caractérisé en ce que l'on prépare un composé de formule Ib dans lequel $R_1$ représente le radical méthyle, éthyle, n-propyle, isopropyle, n-butyle, isobutyle, tert-butyle, pentyle ou hexyle à chaîne droite ou ramifiée, cyclopentyle, cyclohexyle, cycloheptyle, cyclopentényle, cyclohexényle, cycloheptényle, phényle, naphtyle, di- ou tétrahydronaphtyle, benzyle, phénéthyle, p-fluorophénéthyle, o-méthylphénéthyle, p-méthoxyphénéthyle, 2,4-dichlorophénéthyle ou cyclohexyléthyle.

7. Procédé selon la revendication 1 ou 3, caractérisé en ce que l'on prépare un composé de formule Ib dans lequel $R_2$ représente le radical méthyle, éthyle, n-propyle, isopropyle, n-butyle, isobutyle, tert-butyle, pentyle ou hexyle à chaîne droite ou ramifiée, cyclopentyle, cyclohexyle, cycloheptyle, cyclopentényle, cyclohexényle, cycloheptényle, phényle, naphtyle, di- ou tétrahydronaphtyle, benzyle, phénéthyle, p-fluorophénéthyle, o-méthylphénéthyle, p-méthoxyphénéthyle, 2,4-dichlorophénéthyle ou cyclohexyléthyle.

8. Procédé selon l'une des revendications 1 à 7, caractérisé en ce que l'on prépare un composé de formule Ib dans lequel dans lequel les deux ou trois centres chiraux au niveau des atomes de carbone $\alpha$ se présentent en la configuration L.

9. Procédé pour la préparation d'un produit contenant un composé de formule Ib selon l'une des revendications 1 à 8, ou un sel physiologiquement acceptable de celui-ci, caractérisé en ce qu'on le met sous une forme d'administration appropriée.

10. Procédé selon l'une des revendications 1 à 8, pour la préparation d'un composé de formule Ib pour utilisation en tant que médicament.

11. Procédé selon l'une des revendications 1 à 8, pour la préparation d'un composé de formule Ib pour utilisation en tant que médicament dans le traitement de l'hypertension artérielle.